(19) Europäisches Patentamt
European Patent Office
Office européen des brevets

(11) EP 3 090 629 A1

(12) EUROPEAN PATENT APPLICATION

(43) Date of publication:
09.11.2016 Bulletin 2016/45

(51) Int Cl.:
*A01K 67/027* (2006.01) *C12N 15/00* (2006.01)
*C12N 5/0735* (2010.01) *C12N 15/85* (2006.01)

(21) Application number: 16174967.6

(22) Date of filing: 01.02.2006

(84) Designated Contracting States:
AT BE BG CH CY CZ DE DK EE ES FI FR GB GR HU IE IS IT LI LT LU LV MC NL PL PT RO SE SI SK TR

(30) Priority: 01.02.2005 US 49229
15.08.2005 US 204879

(62) Document number(s) of the earlier application(s) in accordance with Art. 76 EPC:
06734219.6 / 1 850 659

(71) Applicant: Synageva BioPharma Corp.
Lexington, MA 02421 (US)

(72) Inventors:
- VAN DE LAVOIR, Marie-Cecile
San Francisco, CA 94127 (US)
- LEIGHTON, Philip A.
San Francisco, CA 94117 (US)

(74) Representative: Dolleymores
9 Rickmansworth Road
Watford, Hertfordshire WD18 0JU (GB)

Remarks:
This application was filed on 17-06-2016 as a divisional application to the application mentioned under INID code 62.

# (54) METHOD FOR PRODUCING TRANSGENIC CHICKENS USING LONG TERM CULTURE OF CHICKEN PRIMORDIAL GERM CELLS

(57) The present invention relates to a method for producing transgenic chickens obtained from long-term cultures of avian PGCs. Also described are techniques to produce said PGCs and transgenic birds derived from prolonged PGC cultures. These PGCs can be transfected with genetic constructs to modify the DNA of the PGC, specifically to introduce a transgene encoding an exogenous protein. When combined with a host avian embryo by known procedures, those modified PGCs are transmitted through the germ line to yield transgenic offspring. Also described are compositions comprising long-term cultures of PGCs that can be genetically modified by gene targeting, that can accept large amounts of foreign DNA and that contribute to the germline of recipient embryos.

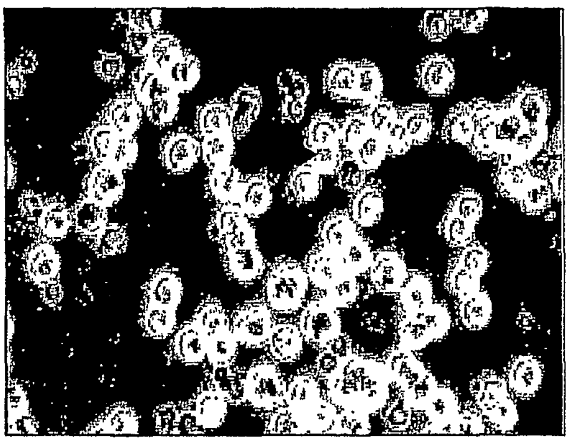

FIGURE 1A

EP 3 090 629 A1

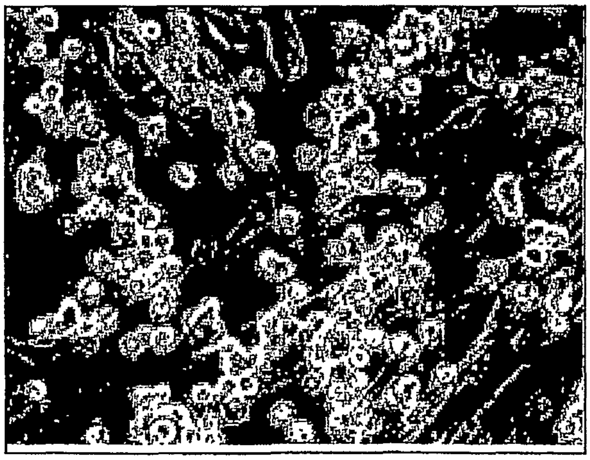

FIGURE 1B

SEQUENCE LISTING

```
<110>  Origen Therapeutics
       Van de Lavoir, Marie-Cecile
       Leighton, Philip

<120>  Transgenic Chickens

<130>  700603.4002EP

<140>  EP06734219.6
<141>  2006-02-01

<150>  US 11/204,879
<151>  2005-08-15

<150>  US 11/049,229
<151>  2005-02-01

<160>  22

<170>  PatentIn version 3.2

<210>  1
<211>  20
<212>  DNA
<213>  Artificial Sequence V-1

<220>
<223>  Primer to amplify a 751 bp fragment from CVH transcript

<400>  1
gctcgatatg ggttttggat                                                    20


<210>  2
<211>  21
<212>  DNA
<213>  Artificial Sequence V-2

<220>
<223>  Primer to amplify a 751 bp fragment from CVH transcript

<400>  2
ttctcttggg ttccattctg c                                                  21


<210>  3
<211>  20
<212>  DNA
<213>  Artificial Sequence Dazl-1

<220>
<223>  Primer to amplify a 536 bp fragment from Dazl transcript

<400>  3
gcttgcatgc ttttcctgct                                                    20


<210>  4
<211>  19
<212>  DNA
<213>  Artificial Sequence Dazl-2
```

<220>
<223>  Primer to amplify a 536 bp fragment from Dazl transcript

<400>  4
tgcgtcacaa agttaggca                                                    19

<210>  5
<211>  21
<212>  DNA
<213>  Artificial Sequence Act-RT-1

<220>
<223>  Primer to amplify a 597 bp fragment from chicken actin transcript

<400>  5
aacaccccag ccatgtatgt a                                                 21

<210>  6
<211>  20
<212>  DNA
<213>  Artificial Sequence Act-RT-2

<220>
<223>  Primer to amplify a 597 bp fragment from chicken actin transcript

<400>  6
tttcattgtg ctaggtgcca                                                   20

<210>  7
<211>  28
<212>  DNA
<213>  Artificial Sequence HS4-Bam-F

<220>
<223>  PCR primer

<400>  7
aggatccgaa gcaggctttc ctggaagg                                          28

<210>  8
<211>  30
<212>  DNA
<213>  Artificial Sequence HS4-Bgl-R

<220>
<223>  PCR Primer

<400>  8
aagatcttca gcctaaagct ttttccccgt                                        30

<210>  9
<211>  136
<212>  DNA
<213>  Artificial Sequence

<220>
<223>  Oligonucleotide Primer for Light Chain V gene

<400> 9
ctctctagac aactcagagt tcaccatgga gaccgatacc ctcctgctat gggtcctcct 60

gctatgggtc ccaggatcaa ccggagatat tcagatgacc cagtctccat ctaccctctc 120

tgctagcgtc ggggat 136

<210> 10
<211> 114
<212> DNA
<213> Artificial Sequence

<220>
<223> Oligonucleotide Primer for Light Chain V gene

<400> 10
ataaattaga agcttgggag ctttgcctgg cttctgctgg taccagtgca tgtaacttat 60

acttgagctg gcagagcagg ttatggtgac cctatccccg acgctagcag agag 114

<210> 11
<211> 123
<212> DNA
<213> Artificial Sequence

<220>
<223> Oligonucleotide Primer for Light Chain V gene

<400> 11
gctcccaagc ttctaattta taccacatcc aacctggctt ctggagtccc tgctcgcttc 60

agtggcagtg gatctgggac cgagttcacc ctcacaatca gctctctgca gccagatgat 120

ttc 123

<210> 12
<211> 127
<212> DNA
<213> Artificial Sequence

<220>
<223> Oligonucleotide Primer for Light Chain V gene

<400> 12
ctcgcgatcg ccaatagtga aaaattacgt ttgacctcca ccttggtccc ctgaccgaac 60

gtgagtgggt aagtactcct ttgatggcag taataagtgg cgaaatcatc tggctgcaga 120

gagctga 127

<210> 13
<211> 95
<212> DNA
<213> Artificial Sequence

<220>
<223> Oligonucleotide Primer for Heavy Chain V gene

<400> 13
ctctcgcgat ctctctgttc acaggcgtgc actctcaggt ccagcttgtc cagtctgggg 60

ctgaagtcaa gaaacctggc tcgagcgtga aggtc 95

<210> 14
<211> 129
<212> DNA
<213> Artificial Sequence

<220>
<223> Oligonucleotide Primer for Heavy Chain V gene

<400> 14
cccagtcgac ggattaatat atccaatcca ttccagaccc tgtccagggg cctgccttac 60

ccagtgcatc ctgtagctag taaaggtgta gccagaagcc ttgcaggaga ccttcacgct 120

cgagccagg 129

<210> 15
<211> 124
<212> DNA
<213> Artificial Sequence

<220>
<223> Oligonucleotide Primer for Heavy Chain V gene

<400> 15
tatattaatc cgtcgactgg gtatactgaa tacaatcaga agttcaagga caaggcaaca 60

attactgcag acgaatccac caatacagcc tacatggaac tgagcagcct gagatctgag 120

gaca 124

<210> 16
<211> 121
<212> DNA
<213> Artificial Sequence

<220>
<223> Oligonucleotide Primer for Heavy Chain V gene

<400> 16
ctctcgcgag gccattctta cctgaggaga ctgtgaccag ggttccttgg ccccagtagt 60

caaagacccc cccccctctt gcacagtaat agactgcggt gtcctcagat ctcaggctgc 120

t 121

<210> 17
<211> 125
<212> DNA
<213> Gallus sp.

<220>
<223> HincII-XbaI fragment upstream of the Ov Kozak translation initiation sequence in the Ovalbumin gene

<400> 17
gttaacattt aattgcctaa aaactgctcg taatttactg ttgtagccta ccatagagta 60

ccctgcatgg tactatgtac agcattccat ccttacattt tcactgttct gctgtttgct 120

ctaga 125

<210> 18
<211> 23
<212> DNA
<213> Artificial Sequence

<220>
<223> Oligonucleotide Primer K8 HincII-F

<400> 18
ggatatagca acagacacat tac 23

<210> 19
<211> 31
<212> DNA
<213> Artificial Sequence

<220>
<223> Oligonucleotide Primer K8-TTT NotIXbaI-R

<400> 19
tttgcggccg ctctagagca aacagcagaa c 31

<210> 20
<211> 125
<212> DNA
<213> Gallus sp.

<220>
<223> 3' OV homology arm located downstream of translation termination codon of ovalbumin gene

<400> 20
aaagaagaaa gctgaaaaac tctgtccctt ccaacaagac ccagagcact gtagtatcag 60

gggtaaaatg aaaagtatgt tatctgctgc atccagactt cataaaagct ggagcttaat 120

ctaga 125

<210> 21
<211> 32
<212> DNA
<213> Artificial Sequence

<220>
<223> Oligonucleotide Primer NotI OV (3'TAA)-F

<400> 21
aaaagcggcc gcaaagaaga aagctgaaaa ac 32

<210> 22
<211> 34
<212> DNA
<213> Artificial Sequence

<220>
<223> Oligonucleotide Primer 3' OVTAA-R2

<400> 22
ctccgcggct cgagtctaga ttaagctcca gctt 34

## Description

RELATED INFORMATION

This invention was made with Government support under USDA SBIR 2003-09058 and NIH R44 GM064261, R43 GM073306-01, R44 HD 039583 and R43 HD 047995-01. The Government has certain rights in this invention.

BACKGROUND OF THE INVENTION

Transgenic animals offer the potential for tremendous advances in the sustainable production of valuable pharmaceutical products, such as antibodies. However, the production of transgenic animals involves significant technical hurdles that have only been overcome for a few species. The ability to incorporate genetic modifications encoding proteins into the DNA of a species for a specific expression requires several distinct technologies that must be developed for each species. One approach to alter the genetic and physical characteristics of an animal is to introduce cells into recipient embryos of the animal. These cells have the ability to contribute to the tissue of an animal bom from the recipient embryo and to contribute to the genome of a transgenic offspring of a resulting animal.

Significant expenditure of time and resources has been committed to the study and development of cell lines, the manipulation of the genome of the cells, and cell culture techniques that permit such engineered cells to be maintained in culture. Although many attempts have been made, the ability to sustain the pluripotency of engineered cells in culture has been achieved for only a few species. If sustainable cell cultures were readily available and susceptible to genetic engineering while maintaining pluripotency, a broad application of new technologies would be available.

In certain cases, the cells can be engineered with a transgene that contains the DNA that encodes an exogenous product such as a protein or an antibody. The transgene contains the blueprint for the production of the protein and contains sufficient coding and regulatory elements to enable the expression of the protein in the tissue of the animal that is created from the insertion of the cells into a recipient embryo. In some circumstances, the expression is desired to be ubiquitous so that the expression occurs in all tissue types. However, in other circumstances, such as the collection of a valuable antibody, the expression must be limited to certain specific tissue types that facilitate collection of the expressed protein. For example, in cows, the expression of a protein in the milk enables the ready collection of the protein by simply collecting the milk of the cow and separating the exogenous protein. In chickens, the robust production of antibodies in the white of the egg also provides an attractive vehicle for the expression and collection of the antibodies. Furthermore, where the tissue specific expression is specific to the oviduct of a chicken, the expression yields antibodies having certain specific desirable chemical properties that increase the therapeutic utility of the antibodies when used in the treatment of a human patient. Thus, one particularly attractive field of research and commercial development is genetically engineered chickens that selectively express antibodies in the egg to facilitate isolation and collection of proteins with desirable chemical properties. For the production of exogenous antibodies, avian biological systems offer many advantages including efficient farm cultivation, rapid growth, and economical production. Further, the avian egg offers an ideal biological design, both for massive synthesis of antibodies and ease of isolation and collection of product. Furthermore, as described below in the context of the present invention, advantages of the transgenic chicken expression system, compared for example to vertebrate, plant, or bacterial cell systems, are readily demonstrated and can be applied to produce uniquely advantageous chemical properties for large quantities of antibody product. The goal of creating a transgenic chicken has been sought by scientists for many, many years. Although the goal has been reached in other species, such as mice, cows, and pigs, transgenic chickens have not been created other than through the use of retroviral technology that suffers from inherent limitations on the size of a transgene that may be introduced into the DNA of the transgenic animal.

However, if a cell culture was sufficiently stable to allow large transgenes to become integrated into the genome of the cell, a transgene encoding tissue specific expression of an antibody can be passed to a transgenic organism by several different techniques depending on the target cell and the specific construct used as the transgene. Whole genomes can be transferred by cell hybridization, intact chromosomes by microcells, subchromosomal segments by chromosome mediated gene transfer, and DNA fragments in the kilobase range by DNA mediated gene transfer (Klobutcher, L.A. and F.H. Ruddle, Annu. Rev. Biochem., 50: 533-554, 1981). Intact chromosomes may be transferred by microcell-mediated chromosome transfer (MMCT) (Foumier, R.E. and F.H. Ruddle, Proc. Natl. Acad. Sci. U.S.A., 74: 319-323, 1977). The specific design of the transgene also must consider the content of the DNA sequences encoding the antibody, the target cell line, the specific tissue in which expression is targeted, the host organism in which expression occurs, and the antibody to be expressed. The transgene designed for tissue specific expression must satisfy several parameters to enable successful integration into the genome of a cell and to insure successful expression in the selected tissue of the host organism.

Insertion of the transgenes that enable tissue specific expression may threaten the pluripotency of the cells unless the transgenes are carefully designed and the culture conditions are optimized. Thus, cell lines suitable for use in transgenesis must be both stable in culture and must maintain pluripotency when the cell is transfected with a genetic construct that is large and complex enough to contain all of the elements necessary for tissue specific and high-level expression where desired. In the resulting transgenic animal, the transgene may optionally be selectively expressed in specific individual tissue types in which the transgene is designed to be expressed. Depending on the genetic content of the transgene, the transgene may not be expressed in other tissues if the viability of the animal or the advantageous chemistry of the resulting protein is compromised.

## SUMMARY OF INVENTION

This invention includes transgenic chickens and technologies enabling genetic engineering of transgenic birds, and the long-term culture of PGCs used to create transgenic chickens. The invention also relates to antibodies produced in chickens and their unique chemistry. Specifically, these antibodies have advantageous chemical properties that enhance their therapeutic utility in certain applications. Antibodies produced in chickens have a distinct pattern of chemical modifications compared to antibodies produced in vertebrate, plant, or bacterial cell systems such that when administered to a patient with the goal of binding a toxin to target tissue, such as tumors, the target tissue is treated with increased therapeutic efficacy. In one embodiment, long term cultures of PCGs are engineered with specially designed genetic constructs to introduce genetic modification into birds, including the insertion of transgenes that yield tissue specific expression of exogenous proteins. Transgenic birds of the invention may also express the transgene-derived antibody in the oviduct and the antibody is deposited in large quantities in the egg. In preferred embodiments, exogenous antibody proteins are encoded by human DNA sequences such that native human antibodies are expressed in the chicken oviduct and may be collected from the egg.

The present invention includes populations of birds exhibiting tissue specific expression of antibodies, transgene constructs that enable exogenous antibody expression, isolated compositions of antibodies produced in chickens and having specially defined chemical properties, and related methods for creation of the birds, production of the antibodies and their therapeutic use in humans. The invention uses long term primordial cell cultures and special techniques to produce chimeric or transgenic birds derived from prolonged cell cultures, wherein the genome of the PGCs have a stably integrated transgene expressing an exogenous protein such that progeny of the cultured cells contain the stably integrated transgene. When introduced to a host avian embryo, by the procedures described below, those modified donor cells produce birds that express the transgene into specific, selected somatic tissue of the resulting animals.

This invention also includes compositions of exogenous proteins expressed in transgenic chickens and having certain desirable chemical properties compared to vertebrate, plant, or bacterial cell systems. Specifically, these proteins, particularly antibodies, have reduced concentrations of fucose, galactose, N-acetyl neuraminic acid, N-glycolylneuraminic acid and elevated concentrations of mannose. Antibodies having some or all of these properties exhibit increased therapeutic utility when administered to a human. Specifically, these antibody compositions exhibit enhanced antibody-dependent cellular cytotoxicity (ADCC). Accordingly, the methods of the invention include using transgenic chickens to enhance the therapeutic utility, based on the ADCC effect, of compositions of antibodies by expressing them in a transgenic chicken.

The invention also includes transgenic chickens expressing exogenous antibody, having the advantageous chemistry defined herein, in the oviduct tissue such that exogenous antibody is concentrated in defined quantities in the egg white. In one preferred embodiment, the exogenous protein is a human sequence monoclonal antibody encoded by the transgene construct incorporated into the genome of a transgenic bird. The human monoclonal antibody encoding polynucleotide sequence is contained within a transgene that is specifically constructed for expression in the oviduct and which contains appropriate promoters and regulatory sequences to facilitate tissue specific expression.

This invention also relates to long-term cultures of avian primordial germ cells (PGCs) and several additional inventions enabled by the creation of a long-term culture where avian PGCs proliferate and where PGC cultures can be extended through multiple passages to extend the viability of the culture beyond 40 days, 60 days, 80 days, 100 days, or longer. The PGCs of the invention proliferate in long term cultures and produce germline chimeras when injected into recipient embryos.

## DESCRIPTION OF THE FIGURES

Figure 1A: PGCs maintained in culture for 54 days. Note that the cells are not attached and maintain a round morphology. Arrows indicate several dividing cells visible in this culture.

Figure 1B: PGCs maintained in culture for 234 days. These cells are cultured on a feeder layer of irradiated STO cells.

Figure 2: Gene expression as determined by RT-PCR of the germ cell markers *CVH* and *Dazl.* Cells were in culture for 32 days. Lane 1 shows expression of both *CVH* and *Dazl* in an aliquot of PGCs. A second sample, in lane 2, did not have sufficient mRNA as determined by the absence of actin. CES cells were also analyzed; actin was expressed but the cES cells did not express *CVH* and *Dazl* was expressed only weakly.

Figure 3: Western analysis of PGCs maintained in culture for 166 days. Testis was used as positive control and liver as a negative control. Rabbit anti-chicken CVH IgG was used as the primary antibody.

Figure 4: Telomeric Repeat Amplification Protocol (TRAP) Assay. Different dilutions of cell extracts of 2 different PGC cell lines (13&16) maintained in culture for 146 days. The positive control consisted of the transformed human kidney cell line 293 and the negative control was lysis buffer only with no template added. In the PGC and positive control lanes, repeat sequences are visible indicating the presence of telomerase.

Figure 5A: cEG cells derived from PGCs maintained in culture. 5B: Chicken embryonic stem cells. Note the small cells, big nucleus (light grey) and pronounced nucleolus in both cell types.

Figure 6: Chimeras obtained from cEG cells derived from PGCs. The EG cells were derived from black feathered Barred Rock embryos. As recipients, a white feathered (White Leghorn) embryo was used. Somatic chimerism is evident by the black feathers.

Figure 7: Rooster IV7-5 with his offspring. A White Leghorn is homozygous dominant at the dominant, white locus (I/I). When bred to a Barred Rock hen (i/i) all offspring from a White Leghorn will be white (I/i). A black chick demonstrates that the injected PGCs (derived from a Barred Rock embryo (i/i)) have entered the germline of the White Leghorn rooster.

Figure 8: Southern analysis of cx-neo transgene in a line of primordial germ cells (PGCs).

Figure 9: FACS analysis of DT40 cells (negative control population), ES cells, EG cells and PGCs, stained with antibodies against chicken vasa homologue (CVH) and 1B3. The DT40, ES and EG cells were negative for both markers while a large majority of PGCs stained for both CVH and 1B3. The cell lines used were PGC 102; ES 439 and EG 455.

Figure 10: Southern analysis of the HS4-β-actin-neo transgene in 2 lines of primordial germ cell PGCs.

Figure 11: Southern blot analysis showing that a clonally-derived, transfected PGC line can contribute to the germline in chimeric chickens and differentiate into EG cells. Upper panel: Genomic DNAs from PGCs transfected with the HS4 bactin-eGFP-bactin-puro construct, three embryos derived from a chimeric rooster made with the transfected PGCs and EG cells derived from the transfected PGCs, were digested with restriction enzymes for detecting internal (*Kpn*I) and junction fragments (*Nco*I, *Afl*II) of the transgene insertion. The digested DNA was separated on a 0.7% agarose gel, blotted to nylon membrane, and probed with radiolabeled eGFP sequences. The sizes of the hybridizing fragments were identical in the PGCs, EG cells, and two embryos that showed green fluorescence (GFP+ embryos). A third, non-fluorescing embryo (WT embryo) showed no hybridization. Lower panel: a schematic of the construct is shown, with the locations of the restriction sites indicated, and the expected restriction fragment sizes shown below. There are two *Kpn*I sites, resulting in a 5.3 kb fragment. *Nco*I and *Afl*II cut within the construct once, and therefore the restriction fragments observed are junction fragments joining the construct with the flanking genomic DNA at the insertion site.

Figure 12: Karyotype of G-09 showing all chromosomes to be diploid. In one copy of GGA 2, the majority of the p arm is either missing or translocated to another chromosome. The other copy of CGA 2 is normal. The cells are ZZ (male).

Figure 13: Section of testes, at 18 days of development, stained with DAPI. GFP positive germ cells are clearly visibly within the seminiferous tubules.

Figure 14: The DAPI stained panel shows a section through a seminiferous tubule of an E18 testis.

Figure 15: Transgenic offspring from a chimera carryings PGCs that are stably transfected with a *β*actin-GFP transgene. The embryos demonstrate that GFP is expressed in all tissues from Stage x (EG&K) to Stage 34 (H&H).

Figure 16: In step 1, the anti- IL-2Rα IgL/IgH cassette is inserted into the Ov BAC by homologous recombination in E. coli by recombineering. The antibody is then under the transcriptional control of the Ov regulatory elements. In step 2, the kanamycin gene used in recombineering is removed by Flp recombinase.

Figure 17 shows the design of light chain V gene oligos designed to anneal to each other to produce the full-length light chain V region as shown. Each oligo is shown with the arrowhead pointing in the 3' direction.

Figure 18 shows the OvBAC construct with 110 kb of sequence 5' of the Ov structural gene, and 30 kb of flanking sequence 3' of the Ov structural gene.

## DETAILED DESCRIPTION OF INVENTION

As used herein, the terms chicken embryonic stem (cES) cells mean cells exhibiting an ES cell morphology and which contribute to somatic tissue in a recipient embryo derived from the area pellucida of Stage X (E-G&K) embryos (the approximate equivalent of the mouse blastocyst). CES cells share several in vitro characteristics of mouse ES cells such as being SSEA-1+, EMA-1+ and telomerase+. ES cells have the capacity to colonize all of the somatic tissues.

As used herein, the terms primordial germ cells (PGCs) mean cells exhibiting a PGC morphology and which contribute exclusively to the germline in recipient embryos, PGCs may be derived from whole blood taken from Stage 12 - 17 (H&H) embryos. A PGC phenotype may be established by (1) the germline specific genes CVH and Dazl are strongly transcribed in this cell line, (2) the cells strongly express the CVH protein, (3) the cells do not contribute to somatic tissues when injected into a Stage X nor a Stage 12-17 (H&H) recipient embryo, (4) the cells give rise to EG cells (see below), or (5) the cells transmit the PGC genotype through the germline when injected into Stage 12-17 (H&H) embryos (Tajima et al. (1993) Theriogenology 40, 509-519; Naito et al., (1994) Mol. Reprod. Dev., 39, 153-161; Naito et al., (1999) J Reprod. Fert. 117, 291-298).

As used herein, the term chicken embryonic germ (cEG) cells means cells derived from PGCs and are analogous in function to murine EG cells. The morphology of cEG cells is similar to that of cES cells and cEG cells contribute to somatic tissues when injected into a Stage X (E-G&K) recipient.

As used herein, the term transgenic means an animal that encodes a transgene in its somatic and germ cells and is capable of passing the transgenic traits to its progeny.

Although the examples herein are described for chickens, other gallinaceous species such as quails, turkey, pheasant, and others can be substituted for chickens under experimentation and with a reasonable expectation for successful implementation of the methods disclosed here.

By inserting DNA constructs designed for tissue specific expression into ES cells in culture, chickens have been created that express valuable pharmaceutical products, such as monoclonal antibodies, in their egg whites. See PCT US03/25270 WO 04/015123 Zhu et al. A critical enabling technology for such animals is the creation and maintenance of truly long-term ES cell cultures that remain viable long enough for the genotype of the cloned cells to be engineered in culture.

Unlike ES cells, however, primordial germ cells (PGCs) have only been cultured on a short-term basis. Once the length in culture extends beyond a short number of days, these cells lose the ability to contribute exclusively to the germline. Typically, PGCs maintained in culture using current culture techniques do not proliferate and multiply. In the absence of robust growth, the cultures are "terminal" and cannot be maintained indefinitely. Over time, these terminal cell cultures are degraded and the cells lose their unique PGC morphology and revert to EG cells. Embryonic germ cells acquire a different morphology from PGCs, lose their restriction to the germline, and gain the ability to contribute to somatic tissues when injected into early stages of embryonic development. To introduce a predetermined genotype into the germline of a recipient embryo, thereby enabling the animal to pass the desired genotype on to future generations, PGCs are uniquely attractive because they are known to be the progenitors of sperm and eggs.

Long-term cultures of PGCs provide several important advantages, such as sustaining valuable genetic characteristics of important chicken breeding lines that are relied upon in the poultry and egg production industries. Currently, extraordinary measures are undertaken to prevent valuable breeding lines from being lost through accident or disease. These measures require maintaining large numbers of members of a line as breeding stock and duplicating these stocks at multiple locations throughout the world. Maintaining large numbers of valuable animals in reserve is necessary, because preserving genetic diversity within a breeding line is also important. By preserving these genetic characteristics in PGC cell cultures, the expense of large scale reserve breeding populations is avoided.

Long term cultures of PGCs are also be highly valuable for the production of pharmaceutical products from the eggs of genetically engineered chickens. Producing genetically engineered chickens using PGCs requires introducing genetic modifications into the genotype of the PGCs while they are maintained in culture. Techniques for a wide variety of genetic manipulations of target cells in culture are well known. However, one main difficulty is that to alter the genotype of PGCs in culture, the culture must remain viable for a length of time adequate to introduce the genetic modifications and to select successfully transformed cells, and while the transfected cells grow and proliferate in culture. Successfully transformed cells that are capable of proliferating are distinguished by their ability to generate large numbers of cells (e.g. $10^4$ to $10^7$ cells) within several days to several weeks following clonal or nearly clonal derivation. The founder cells will be the rare cells that carry the genetic modification that is desired. Typically, these cells are generated in culture at frequencies of $10^{-4}$ to $10^{-7}$ following the application of technologies for genetic modification that are well known, (e.g. lipofection or electroporation). Therefore, production of cells in culture requires passaging the cells to provide space and nutrients for the cells to proliferate and generate a sufficient number of cells to allow selection of the rare, genetically-modified cells in culture.

In addition, the culture conditions must be sufficiently robust to allow the cells to grow from an individual genetically-modified cell into a colony of $10^4$ to $10^7$ cells to be used for genetic analysis in vitro and for the production of chimeras. Thus, if the length of the culture could be extended while preserving the genotype and phenotype of the cells as true PGCs, the cells could be engineered and introduced into recipient embryos at a point in embryonic development when the germline competent cells are migrating to the gonad. These engineered PGCs would contribute exclusively to the nascent population of spermatogonia or oogonia (i.e., the sperm and eggs) in the resulting animals upon maturity. In such a resulting animal, the entirety of the somatic tissue would be derived from the recipient embryo and the germline would contain contributions from both the donor cells and the recipient embryos. Because of the mixed contribution to the germline, these animals are known as "germline chimeras." Depending on the extent of chimerism, the offspring of germline chimeras will be derived either from the donor cell or from the recipient embryo.

The germline in chickens is initiated as cells from the epiblast of a Stage X (E-G & K) embryo ingress into the nascent hypoblast (Kagami et al., (1997) Mol Reprod Dev 48, 501-510; Petitte, (2002) J Poultry Sci 39, 205-228). As the hypoblast progresses anteriorly, the pre-primordial germ cells are swept forward into the germinal crescent where they can be identified as large glycogen laden cells. The earliest identification of cells in the germline by these morphological criteria is approximately 8 hours after the beginning of incubation (Stage 4 using the staging system established by Hamburger and Hamilton, (1951) J Morph 88,49-92). The primordial germ cells reside in the germinal crescent from Stage 4 (H&H) until they migrate through the vasculature during Stage 12-17 (H&H). At this time, the primordial germ cells are a small population of about 200 cells. From the vasculature, the primordial germ cells migrate into the genital ridge and are incorporated into the ovary or testes as the gonad differentiates (Swift, (1914) Am. J. Anat. 15, 483 - 516; Meyer, (1964) Dev Biol. 10,154-190; Fujimoto et al. (1976) Anat. Rec. 185,139 - 154).

In all species that have been examined to date, primordial germ cells have not proliferated in culture for long periods without differentiating into EG cells. Long periods in culture are required in order to produce a sufficient number of cells to introduce a genetic modification by conventional electroporation or lipofection protocols. Typically, these protocols require $10^5$ to $10^7$ cells and therefore, production of these cells from a single precursor requires 17 to 24 doublings assuming that all cell divisions are (1) synchronous and (2) produce two viable daughter cells. The introduction of a genetic modification into the genome of a cell is a rare event, typically occurring in one in $1 \times 10^4$ to $1 \times 10^6$ cells. Following genetic modification, the cells must be able to establish a colony from the single cell that carries and/or expresses the genetic modification. The colony must be able to expand into a population of $10^5$ to $10^7$ cells that can be analyzed by PCR or Southern analysis to evaluate the fidelity of the transgene and provide a sufficient number of cells that are then injected into recipient Stage 13-15 (H&H) embryos. Therefore another 17 to 24 cell divisions are required to produce the populations of cells and in total 34 to 58 doublings are required to produce the population of genetically modified cells. Assuming that the cell cycle is 24 hours, a minimum of 34 days and in general 58 days in culture are required to produce genetically modified primordial germ cells for injection into Stage 13-15 (H&H) recipient embryos. The injected cells must then be able to colonize the germline, form functional gametes and develop into a new individual post fertilization.

Several attempts to establish long-term culture cell lines of chicken PGCs have been reported but none of these attempts has yielded a line of cells that could be sustained. In each of these cases, the culture of PGCs has differentiated into EG cells <u>See</u> WO 00/47717; WO99/06533; WO99/06534; Park et al., (2003) Mol. Reprod. Dev. 65, 389-395; Park and Han, (2000) Mol. Reprod. Dev. 56, 475-482, or cells with an ES cell phenotype, <u>See</u> WO 01/11019. In other cases, PGC cultures could be maintained for only 5 days (Chang et al., (1997) Cell Biology International 21, 495-499; Chang et al., (1995) Cell Biology International 19, 569 - 576) or 10 days (Park et al., (2003) Biol. Reprod. 68, 1657 - 1662). In another case, PGCs were maintained in culture for 2 months, but the cells proliferated only very slowly and the culture could not be passaged (Han et al., (2002) Theriogenology 58, 1531-1539). The ability of a PGC culture to be passaged is a critical property of a long-term culture used for genetic modification of PGCs and for most valuable agricultural and breeding technologies.

The ability of PGC cell cultures to proliferate is essential for selection of cells whose genome has been altered by random integration of a transgene or by site-specific modification. In both types of genetic modification, the proportion of cells acquiring the genetic modification as a stable integration into the genome of the cell in culture is very low on the order of one cell in between ten thousand and one hundred million (i.e. $1 \times 10^{-4}$ to $1 \times 10^{-8}$). Accordingly, the ability to establish a rapidly growing culture is required to obtain an adequate population of cells derived from the rare event that creates the genetic modification in the genome of a cell in culture.

Chicken primordial germ cells have been genetically modified using a retroviral vector within a few hours following isolation from Stage 11-15 embryos (Vick et al., (1993) Proc. R. Soc. Lond. B 251, 179 - 182). However, the size of the transgene is generally limited to less than about 15 kb, usually less than 10 kb and most commonly less 8 kb and site-specific changes to the genome cannot be created using this technology. Stable genetic modifications requiring the insertion of greater than 15kb of exogenous DNA into the genome of cultured avian PGCs have not been previously reported.

Any limitation on the size of an exogenous DNA transgene that may be stably introduced in a long-term PGC cell culture is a critical constraint on the ability to achieve valuable genetic modifications in the genome of PGCs in culture, and in turn, limits the types of genetic modifications that may be passed through germline to offspring of the recipient embryo. For example, the introduction of exogenous DNA encoding a protein into the genome of a transgenic chicken is a highly desirable genetic modification. If a flock of such transgenic chickens could be created, large quantities of valuable proteins could be expressed in the chicken and collected in the egg. The avian egg offers an ideal repository for biologically active proteins and provides a convenient milieu from which proteins can be isolated. Avian animals are also attractive candidates for a broad variety of transgenic technologies. However, application of the full range of mammalian transgenic techniques to avian species has been unsuccessful due to the absence of a cultured cell population into which genetic modifications can be introduced and transmitted into the germline. In a recent paper, Sang et al. state: "It is unlikely that PGCs can be maintained in culture and proliferate for the extended period necessary to identify gene targeting events without losing their ability to migrate to the developing gonad after transfer." Prospects for Transgenesis in the Chick, Mechanisms of Development, 121, 1179-1186, (2004). Therefore, to date, genetically transfected PGCs have not been created and the transmission to a mature living animal of a genetic modification introduced into an avian PGC has not been demonstrated.

Primordial germ cells (PGCs) are the precursors of sperm and eggs and are segregated from somatic tissues at an early stage of development in most animals. Pursuant to this invention, chicken PGCs are isolated, cultured and genetically modified while maintaining their commitment to the germline. In addition, PGCs are induced to differentiate into embryonic germ cells (EGCs), which resemble chicken embryonic stem cells (ESCs) in their commitment to somatic tissues. These PGCs commit to somatic tissues and the germline and provide a unique resource for genetic modification of the genome in chickens.

The PGCs maintained in the culture described herein maintain a characteristic PGC morphology while maintained in culture. The PGC morphology may be observed by direct observation, and the growth of cells in culture is assessed by common techniques to ensure that the cells proliferate in culture. Cell cultures that proliferate are defined as non-terminal and are observed to have a greater number of cells in culture at the latter of 2 distinct time points. The PGCs in the culture of the invention may have 1 X $10^5$ or more cells in any particular culture and this number may be observed to increase over time. Accordingly, the invention includes a proliferating PGC culture that contains a larger number of cells. after a period of days, weeks, or months compared to an earlier time point in the life of the culture. Ideally, the culture contains at least 1 X $10^5$ cells and may be observed to have a higher number after any length of time growing in culture. Furthermore, the PGCs may be observed to be the dominant species in the culture such that, when considering the minimal contribution made by non-chicken feeder cells, the proliferating component of the cell culture consists essentially of chicken primordial germ cells, to the substantial exclusion of other chicken-derived cells.

The culture also manifests the characteristic of allowing proliferation by passage such that samples or aliquots of cells from an existing culture can be separated and will exhibit robust growth when placed in new culture media. By definition, the ability to passage a cell culture indicates that the cell culture is growing and proliferating and is non-terminal. Furthermore, the cells of the invention demonstrate the ability to create germline chimeras after several passages and maintain a PGC morphology. As described herein, this proliferation is an essential feature of any cell culture suitable for stable integration of exogenous DNA sequences.

The PGCs of the invention can be obtained by any known technique and grown in the culture conditions described herein. However, it is preferred that whole blood is removed from a stage 15 embryo and is placed directly in the culture media described below. This approach differs from other approaches described in the literature wherein PGCs are subjected to processing and separation steps prior to being placed in culture. Unlike conventional culture techniques, the culture and methodology of the present invention relies on robust differential growth between PGCs and other cells from whole blood that may initially coexist in the medium, in order to provide the large populations of PGCs in culture described here. Accordingly, the present invention provides culture of PGCs derived directly from whole blood that grow into large cell concentrations in culture, can go through an unlimited number of passages, and exhibit robust growth and proliferation such that the PGCs in culture are essentially the only cells growing and proliferating. These culture conditions provide an important advantage of the present invention, thereby rendering the collection, storage, and maintenance of PGCs in culture particularly simple and efficient and providing a readily available source of donor cells to create germline chimeras that pass the genotype of cultured PGC cells to offspring.

The PGCs maintained in culture by the inventors have demonstrated the existence of a non-terminal culture and have currently existed for at least 327 days in culture. These cells are growing and proliferating in the same manner as was observed at 40, 60, 80, or 100 days (and all integral values therein) and the cells continue to contribute to germline chimeras as described below, and thus, exhibit the primary distinguishing characteristics of true PGCs, i.e., the exclusive contribution to the germline when introduced into a recipient embryo. The culture methodology of the invention includes using whole blood, which contains red blood cells and other metabolically active cell types, placing a mixture of cells into culture along with primordial germ cells and allowing the culture to evolve to consist essentially of avian PGCs displaying the long-term culture characteristics described herein. Cell culturing technology described herein avoids any cell separation processes or techniques and relies solely on differential growth conditions to yield the predominance of PGCs in culture. The use of whole blood as the source of the established and cultured PGC cells offers practical advantages in the efficiency and utility of establishing the cultures and using the cells for agricultural or transgenic purposes. Accordingly, in one aspect of the invention, the culture medium is conditioned with BRL (Buffalo Rat Liver cells), contains fibroblast growth factors, stem cell factor, and chicken serum. The particular characteristics of the medium are described in greater detail below.

In one aspect of the present invention, a culture is established that has a large number of PGCs that are genetically identical and which proliferate to yield a long-term cell culture. These PGCs can be used repeatedly to create germline chimeras by introducing the PGCs from a proliferating long-term culture to recipient embryos. In previous attempts to use PGCs to create germline chimeras, the number of chimeras that could be created was inherently limited by the inability to grow long-term cultures of true PGCs that retain the PGC phenotype. Because long-term cultures are enabled by the present invention, any number of germline chimeras can be created from the same cell culture and an entire population of germline chimeras can be established having genetically identical, PGC-derived germlines. Accordingly, one aspect of the present invention is the creation of large numbers, including greater than 3, greater than 4, greater than 5, 10, 15 and 20 germline chimeric animals all having genetically identical PGC-derived cells in their germline. Another aspect of the invention is the creation of a population of germline chimeras having genetically identical PGC-derived cells in their germline that have, within the population, age differentials that reflect the use of the same long-term cell culture to create germline chimeras. The age differentials exceed the currently available ability to culture primordial germ cells over time and are as high as 190 days without freezing. Accordingly, the present invention includes two or more germline chimeras having identical PGC-derived cells in their germline that differ in age by more than 40 days, 60 days, 80 days, 100 days, 190 days, etc., or any other integral value therein-without freezing the cells. The invention also includes the existence of sexually mature germline chimeras having genetically identical PGC-derived cells in their germline, together with the existence of a non-terminal PGC culture used to create these germline chimeras and from which additional germline chimeras can be created.

Because the PGCs can be maintained in culture in a manner that is extremely stable, the cells can also be cryo-preserved and thawed to create a long-term storage methodology for creating germline chimeras having a capability to produce offspring defined by the phenotype of the PGCs maintained in culture.

The capability to produce large numbers of germline chimeras also provides the ability to pass the PGC-derived genotype through to offspring of the germline chimera. Accordingly, the present invention includes both populations of germline chimeras having genetically identical PGC-derived cells in the germline, but also offspring of the germline chimeras whose genotype and phenotype is entirely determined by the genotype of the PGCs grown in culture. Transmission of a PGC-derived phenotype through the germline has been observed for more than 20 birds at transmission percentages as high as 86%. Thus, the invention includes the offspring of a germline chimera created by germline transmission of a genotype of a primordial germ cell held in culture. Accordingly, the invention includes each of the existence of a primordial germ cell culture containing PGCs of a defined phenotype, a germline chimera having the same primordial germ cells as part of its germline, and an offspring of the germline chimera having a genotype and phenotype dictated by the PGCs in culture.

As has been described previously, the existence of long-term PGC cultures enables the ability to stably transfect the cells in culture with DNA encoding exogenous proteins or introducing other desirable genetic manipulations such as gene insertions and knock-outs of a transgenic animal. Accordingly, all of the above-described populations of PGCs in culture, germline chimeras, and offspring of germline chimeras can also be comprised of a DNA construct stably integrated into the genome of the primordial germ cell, transmitted into the germline of the germline chimera, and transmitted into future generations comprised of offspring of the germline chimeras.

The primordial germ cells may contain virtually any engineered genetic constructs and may be used to introduce genetic modifications into birds that exceed the size restriction currently imposed by retroviral technologies, including the site-specific modifications to the genome and/or insertion of transgenes encoding full length exogenous proteins such as monoclonal antibodies. In a preferred embodiment, genetically engineered chickens express exogenous proteins in a tissue specific fashion in the oviduct to express exogenous proteins in the egg.

The PGC cultures of the invention are sufficiently stable to allow a transgene to become stably integrated into the genome of the PGC, to isolate the genetically modified cells from non-modified cells in the culture, and to introduce the modified cells into a recipient embryo, while maintaining the ability of the cultured PGCs to contribute to the germline in a resulting chimera. In cases where expression of the transgene is controlled by a tissue specific promoter, the transgene would not be expressed in PGCs. In these cases, the transgene would be expressed in the selected tissues in transgenic offspring of the germline chimera. Whole genomes can be transferred by cell hybridization, intact chromosomes by microcells, subchromosomal segments by chromosome mediated gene transfer and DNA fragments in the kilobase range by DNA mediated gene transfer (Klobutcher, L.A. and F.H. Ruddle, Ann. Rev. Biochem., 50: 533-554, 1981). Intact chromosomes may be transferred by microcell-mediated chromosome transfer (MMCT) (Foumier, R.E. and Ruddle, F.H., Proc. Natl. Acad. Sci., USA 74: 319-323, 1977).

Stable long-term cultures of PGCs that yield genetically engineered chickens are necessary for several applications in avian transgenesis, including the production of proteins for the pharmaceutical industry, production of chickens that deposit human monoclonal antibodies in their eggs, and to make site-specific changes to the avian genome for any number of other applications including human sequence polyclonal antibodies.

The ration of donor-derived and recipient-derived PGCs in a recipient embryo can be altered to favor colonization of the germline in PGC-derived chimeras. In developing chicken and quail embryos, exposure to busulfan either greatly reduces or eliminates the population of primordial germ cells as they migrate from the germinal crescent to the gonadal ridge (Reynaud (1977a) Bull Soc. Zool. Francaise 102, 417-429; Reynaud (1981) Arch Anat. Micro. Morph. Exp. 70, 251 - 258; Aige-Gil and Simkiss (1991) Res. Vet. Sci. 50, 139 - 144). When busulfan is injected into the yolk after 24 to 30 hours of incubation and primordial germ cells are re-introduced into the vasculature after 50 to 55 hours of incubation, the germline is repopulated with donor-derived primordial germ cells and subsequently, donor derived gametes are produced (Vick et al. (1993) J. Reprod. Fert. 98, 637 - 641; Bresler et al. (1994) Brit. Poultry Sci. 35 241- 247).

Methods of the invention include: obtaining PGCs from a chicken, such as from the whole blood of a stage 15 embryo, placing the PGCs in culture, proliferating the PGCs to increase their number and enabling a number of passages, creating germline chimeras from these long-term cell cultures, and obtaining offspring of the germline chimeras having a genotype provided by the cultured PGCs. The methods of the invention also include inserting genetic modifications into a population of PGCs in culture to create stably transfected PGCs, selecting cells from this population that carry stably integrated transgenes, injecting the genetically modified cells carrying the stably integrated transgenes into a recipient embryo, developing the embryo into a germline chimera containing the genetic modification in the germline, raising the germline chimera to sexual maturity and breeding the germline chimera to obtain transgenic offspring wherein the genetic modification is derived from the cultured PGC.

The following describes the unexpected finding that PGCs can be isolated from the blood of Stage 12-17 (H&H) embryos, that the cells will proliferate rapidly and maintain their PGC phenotype, that the PGCs can be passaged at daily or 2-day or 3-dayintervals, that the PGCs will colonize the germline but not somatic tissues after at least 110 days in culture, that viable offspring can be obtained from cells that have been in culture for 110 days, that the PGCs can be genetically modified by transfection with a transgene, and that the genetically modified PGCs can be isolated and propagated into a colony of genetically modified PGCs.

Pursuant to this invention, chicken PGC cell lines have been derived from blood taken from Stage 14-16 (H&H) embryos that have a large, round morphology (Figure 1). These cells are confirmed to be chicken PGCs by their morphology after long term culturing and their ability to yield PGC-derived offspring. In addition, the PGC cultures express the germline-specific genes Dazl and CVH (Figure 2) and the CVH protein is produced by the cells in culture (Figure 3). PGCs in culture also express telomerase (Figure 4) indicating that they have an immortal phenotype. Furthermore, PGCs will give rise to embryonic germ (EG) cells in the appropriate culture conditions (Figure 5). By analogy, mouse and human PGCs will give rise to EG cells when treated in an analogous fashion. Mouse EG cells will contribute to somatic tissues and chicken EG cells also contribute to somatic tissues as indicated by black feather pigmentation in chimeras (Figure 6). Chicken PGCs have been genetically modified as indicated by Southern analysis (Figure 7). In this embodiment, the CX promoter is stably integrated into the genome of a PGC and is used to facilitate expression of the gene encoding aminoglycoside phosphotransferase (APH) which is also integrated into the genome of a PGC and is used to confer resistance to neomycin added to culture media in order to select PGCs that have been genetically modified.

Example 1. Derivation of cultures of chicken PGCs

Two to five $\mu$L of blood taken from the sinus terminalis of Stage 14 - 17 (H&H) embryos were incubated in 96 well plates in a medium containing Stem Cell Factor (SCF; 6 ng/ml or 60 ng/ml), human recombinant Fibroblast Growth Factor (hrFGF; 4ng/ml or 40 ng/ml), 10% fetal bovine serum, and 80% KO-DMEM conditioned medium. Preferably one to three $\mu$L was taken from the vasculature of a stage 15-16 (H&H) embryo. The wells of the 96-well plates was seeded with irradiated STO cells at a concentration of 3 x $10^4$ cells/$cm^2$.

KO-DMEM conditioned media were prepared by growing BRL cells to confluency in DMEM supplemented with 10% fetal bovine serum, 1% pen/strep; 2mM glutamine, 1mM pyruvate, 1X nucleosides, 1X non-essential amino acids and 0.1mM ß-mercaptoethanol and containing 5% fetal bovine serum for three days. After 24 h, the medium was removed and a new batch of medium was conditioned for three days. This was repeated a third time and the three batches were combined to make the PGC culture medium.

After approximately 180 days in culture, one line of PGCs was grown in media comprised of 40% KO-DMEM conditioned media, 7.5% fetal bovine serum and 2.5% chicken serum. Under these conditions, the doubling time of the PGCs was approximately 24-36 hours.

When the culture was initiated, the predominant cell type was fetal red blood cells. Within three weeks, the predominant cell type was that of a PGC. Two PGC cell lines were derived from 18 cultures that were initiated from individual embryos.

A line of PGCs has been in culture for over 9 months, maintain a round morphology, and remain unattached (Figures 1A &B). PGCs have been successfully thawed after cryopreservation in $CO_2$ independent medium containing 10% serum and 10% DMSO.

Example 2. Cultured PGCs express CVH and Dazl

Expression of *CVH,* which is the chicken homologue of the germline specific gene *VASA* in Drosophila, is restricted to cells within the germline of chickens and is expressed by approximately 200 cells in the germinal crescent (Tsunekawa et al., 2000). *CVH* expression is required for proper function of the germline in males; loss of *CVH* function causes infertility in male mice (Tanaka et al., 2000). The expression of *Dazl* is restricted to the germline in frogs (Houston and King, 2000) axolotl (Johnson et al., 2001), mice (Schrans-Stassen et al., 2001), rat (Hamra et al., 2002), and human (Lifschitz-Mercer et al., 2000). Deletion of *Dazl* led to spermatogenic defects in transgenic mice (Reijo et al., 1995).

After 32 days, PGCs were washed with PBS, pelleted and mRNA was isolated from the tissue samples with the Oligotex Direct mRNA kit (Qiagen). cDNA was then synthesized from 9 μl of mRNA using the SuperScript RT-PCR System for First-Strand cDNA synthesis (Invitrogen). Two μl of cDNA was used in the subsequent PCR reaction. Primer sequences which were derived from the *CVH* sequence (accession number AB004836), *Dazl* sequence (accession number AY211387), or β-actin sequence (accession number NM_205518) were :

| | |
|---|---|
| V-1 | GCTCGATATGGGTTTTGGAT (SEQ ID NO.1) |
| V-2 | TTCTCTTGGGTTCCATTCTGC (SEQ ID NO.2) |
| Dazl-1 | GCTTGCATGCTTTTCCTGCT (SEQ ID NO.3) |
| Dazl-2 | TGC GTC ACA AAG TTA GGC A (SEQ ID NO.4) |
| Act-RT-1 | AAC ACC CCA GCC ATG TAT GTA (SEQ ID NO.5) |
| Act-RT-2 | TTT CAT TGT GCT AGG TGC CA (SEQ ID NO.6) |

Primers V-1 and V-2 were used to amplify a 751 bp fragment from the *CVH* transcript.

Primers Dazl-1 and Dazl-2 were used to amplify a 536 bp fragment from the *Dazl* transcript.

Primers Act-RT-1 and Act-RT-R were used to amplify a 597 bp fragment from the endogenous chicken β-actin transcript. PCR reactions were performed with AmpliTaq Gold (Applied Biosystems) following the manufacturer's instructions.

Example 3. PGCs express the CVH rotein

Protein was extracted from freshly isolated PGCs using the T-Per tissue protein extraction kit (Pierce). Protein from cells was extracted by lysing the cells in 1% $NP_4O$; 0.4% deoxycholated 66mM EDTA; 10mM,Tris, pH7.4. Samples were run on 4-15% Tris-HCL ready gel (Bio-Rad). After transfer onto a membrane, Western blots were performed with Super Signal West Pico Chemiluminescent Substrate kits (Pierce) as instructed. A rabbit anti-CVH antibody was used as a primary antibody (1:300 dilution) and a HRP-conjugated goat anti-rabbit IgG antibody (Pierce, 1:100,000) was used as a secondary antibody (Figure 3).

Example 4. Cultured PGCs express telomerase

Primordial germ cells were pelleted and washed with PBS before being frozen at-80°C until analysis. Cell extracts were prepared and analyzed according to the manufacturer's directions using the TRAPeze Telomerase Detection Kit (Serologicals Corporation) which is based upon the Telomeric Repeat Amplification Protocol (TRAP) (Kim et al., 1994). Figure 4.

Example 5. Embryonic germ (EG) cells can be derived from cultures of PGCs

Chicken EG cells have been derived from PGCs by allowing the cells to attach to the plate , removing FGF, SCF and chicken serum and to culture the cells under the same conditions used for ES cell culture (van de Lavoir et al., 2006 High Grade Somatic Chimeras from Chicken Embryonic Stem Cells, Mechanisms of Development 12, 31-41; van de Lavoir and Mather-Love (2006) Chicken Embryonic Stem Cells; Culture and Chimera Production, Methods in Enzymology, in press). The morphology of the cEG cells is very similar to that of the cES cells (Figure 5A,B). When cEG cells are injected into Stage X (E-G&K) embryos, they have the ability to colonize somatic tissues and make chimeras that, as juveniles, appear identical to chimeras made with cES cells (Figure 6). Chicken EG cells are observed in both newly derived and clonally derived transgenic PGC lines. Southern analysis of EG cells derived from GFP positive PGCs (Figure 11) demonstrate that EG cells originate from the PGCs

Example 6. Cultured male PGCs give rise to functional gametes in roosters

Male primordial germ cell lines were derived from individual Barred Rock embryos. After establishment of the line, the cells were injected into Stage 13-15 (H&H) embryos. Phenotypically, the hatched chicks resembled White Leghorns. The males were reared to sexual maturity and have been mated to Barred Rock hens (Table 1). Black offspring were indicative of germline transmission of the injected PGCs. The rate of germline transmission of the roosters varied from <1% to 86 % (Table 1).

Table 1: Germline transmission of male primordial germ cells injected into the vasculature of Stage 14-15 (H&H) embryos.

| Cell line | Sex | Age | # cells injected | # Roosters tested | germline transmission* |
|---|---|---|---|---|---|
| PGC13 | M | 40 | 1200 | 3 | 0.1,1.5,17 |
| | | 110 | 2500-3000 | 5 | 1,1,1.5,3,84 |
| PGC21 | M | 44 | 1500 | 3 | 10,16,21 |
| PGC34 | M | 47 | 3000 | 3 | 42,74,80 |
| PGC35 | M | 35 | 3000 | 7 | 15,23,47,61,80,85,86 |
| PGC51 | M | 47 | 3000 | 1 | 11 |
| PGC54 | M | 47 | 3000 | 4 | 0.5,2,20,24 |
| PGC80 | M | 29 | 3000 | 1 | 55 |
| PGC84 | M | 50 | 3000 | 1 | 70 |
| *Each value represents the rate of germline transmission of one chimera | | | | | |

PGCs may also be injected into the subgerminal cavity of stage X embryos. 1000 or 5000 PGCs were injected after 209 days of culture into irradiated embryos. Hatched male chicks were grown to sexual maturity and bred to test for germline transmission. In 3 out of 4 roosters tested germline transmission observed in varying frequency of 0.15 to 0.45%. This indicates that PGCs can colonize the germline when injected before gastrulation. Germline transmission of male PGCs has not been observed in 1,625 offspring of 14 female chimeras.

Example 7. Cultured female PGCs give rise to functional gametes in hens

Female PGCs from Barred Rock embryos that were cultured 66 days were injected into Stage 13 - 16 (H&H) White Leghorn embryos and all hatched chicks were phenotypically White Leghorns. The hens were reared to sexual maturity and have been mated to Barred Rock roosters (Table 1). Female PGCs transmitted through female chimeras at frequencies up to 69%. (Table 1).

Table 2. Germline transmission of female primordial germ cells injected into the vasculature of Stage 14-15 (H&H) embryos.

| Cell line | Sex | Age | # cells injected | # hens tested | germline transmission* |
|---|---|---|---|---|---|
| PGC56 | F | 66 | 3000 | 5 | 1,2,6,52,69 |
| PGC85 | F | 47 | 3000 | 10 | 0,0,0,2,2,4,5,10,11,12 |
| *Each value represents the rate of germline transmission of one chimera | | | | | |

Female PGCs were also injected into male recipient White Leghorn embryos. The male chimeras were reared to sexual maturity and bred to Barred Rock hens. Germline transmission of female PGCs was not observed in 506 offspring of three roosters tested.

Example 8. Offspring derived from PGCs are reproductively normal

Three male and 4 female non-transgenic PGC derived offspring were bred together. Between 53 and 100 % of the eggs were fertile (Table 3) and between 79 and 100 % of the fertile eggs resulted in a hatched embryo (Table 3), indicating that PGC derived offspring are reproductively normal.

Table 3. Reproductive parameters of PGC offspring obtained from germline chimeric roosters.

| Rooster | Hen | Eggs set | Infertile/ early dead | Fertility % | # Hatched | % hatched of fertile embryos |
|---|---|---|---|---|---|---|
| IV9-1-7 & IV9-1-8 | IV9-1-1 | 36 | 17 | 53 | 15/19 | 79 |
| IV9-1-2 & IV9-1-8 | IV9-1-4 | 33 | 5 | 85 | 27/28 | 96 |
| IV9-1-7 & IV9-1-8 | IV9-1-5 | 38 | 8 | 79 | 28/30 | 93 |
| IV9-1-2 | IV9-1-6 | 12 | 0 | 100 | 12/12 | 100 |

Example 9. Sensitivity of PGCs to neomycin and puromycin

The sensitivity of PGCs to puromycin and neomycin was determined to establish the concentration of puromycin and neomycin required to allow the growth of cells that express antibiotic resistance under the control of the CX-promoter which is strongly expressed in all tissues (Origen Therapeutics, unpublished). These experiments demonstrated that a concentration of 300 $\mu$g/ml neomycin for 10 days is necessary to eliminate all non-transfected cells. A concentration of 0.5$\mu$g/ml puromycin was sufficient to eliminate PGCs within 7-10 days.

Example 10. Genetic modification of PGCs

Twenty microgram (20$\mu$l) of a NotI linearized cx-neo transgene (see Fig 8) was added to a population of 5.8x$10^6$ PGCs that had been in culture for 167 days. The cells and DNA were resuspended in 800 $\mu$l of electroporation buffer and 8 square wave pulses of 672 volts and 100 $\mu$sec duration were applied. After ten minutes, the cells were resuspended in culture medium and aliquoted into 24-well plates. Two days after electroporation, 300 $\mu$g of neomycin were added per ml of medium to select cells that were expressing the cx-neo transgene. The cells were kept under selection for 19 days. After 19 days, the cells were taken off selection and expanded for analysis. A proportion of the PGCs was kept under 300$\mu$g/ml for another 31 days demonstrating that the PGCs were functionally resistant to the antibiotic.

Referring to Figure 8, for the plasmid control, the cx-neo plasmid DNA was linearized with NotI and then digested with EcoRI or BamHI to produce a fragment that is slightly smaller (5 kb) than the intact plasmid which is shown by the HindIII digestion. Internal fragments of approximately 2 kb of the cx-neo plasmid were released by digestion with StyI or NcoI. A larger internal fragment of approximately 2.6 kb was released by digestion with EcoRI and KpnI. Digestion of genomic DNA from the line of PGCs with EcoRI, BamHI and HindIII revealed bands that are larger than 6 kb illustrating that the cx-neo transgene was incorporated into the PGC genome. The internal fragments revealed in plasmid DNA following digestion with StyI, NcoI and EcoRI with KpnI were also present in genomic DNA from the PGCs indicating that the cx-neo transgene was integrated into the PGC genome without alteration. Using conventional transgene construction techniques, additional elements can be incorporated such as regulatory elements, tissue specific promoters and exogenous DNA encoding proteins are examples. Monoclonal antibodies are preferred example of a protein encoded by exogenous DNA and human monoclonals are preferred species thereof.

As noted above, the performance of genetic modifications in PGCs to produce transgenic animals has been demonstrated in only a very few species. Analogous genetic manipulations can be achieved in chicken PGCs by referring to those achieved using ES cells in mice. In mice, the separate use of homologous recombination followed by chromosome transfer to embryonic stem (mES) cells for the production of chimeric and transgenic offspring is well known. Powerful techniques of site-specific homologous recombination or gene targeting have been developed (see Thomas, K.R. and M.R. Capecchi, Cell 51: 503-512, 1987; review by Waldman, A.S., Crit. Rev. Oncol. Hematol. 12: 49-64, 1992). Insertion of cloned DNA (Jakobovits, A., Curr. Biol. 4: 761-763, 1994) and manipulation and selection of chromosome fragments by the Cre-loxP system techniques (see Smith, A.J. et al., Nat. Genet. 9:376-385, 1995; Ramirez-Solis, R. et al., Nature 378:720-724, 1995; US Patents 4,959,317; 6,130,364; 6,130,364; 6,091,001; 5,985,614) are available for the manipulation and transfer of genes into mES cells to produce stable genetic chimeras. Many such techniques that have proved useful in mammalian systems would be available to be applied to chicken PGCs if the necessary cultures were available.

The genome of primordial germ cells is generally believed to be in a quiescent state and therefore the chromatin may be in a highly condensed state. Extensive testing of conventional electroporation protocols suggest that special methods are needed to introduce genetic modifications into the genome of PGCs. As described below, the transgenes may be surrounded with insulator elements derived from the chicken β-globin locus to enhance expression. The inclusion of the β-globin insulator elements routinely produces clones that can be grown, analyzed and injected into recipient embryos.

The conventional promoters that are used to drive expression of antibiotic (e.g. neomycin, puromycin, hygromycin, his-D, blasticidin, zeocin, and gpt) resistance genes are expressed ubiquitously. Typically, the promoters are derived from "housekeeping" genes such as β-actin, CMV, or ubiquitin. While constitutive promoters are useful because they are typically expressed at high levels in all cells, they continue to be expressed in most if not all tissues throughout the life of the chicken. In general, expression should be limited to only the tissue and stage of development during which expression is required. For selection of primordial germ cells, the period during which expression is required is their residence in vitro when the antibiotic is present in the media. Once the cells have been inserted into the embryo, it is preferable to terminate expression of the selectable marker (i.e. the antibiotic resistance gene). To restrict expression of the antibiotic resistance genes, the "early response to neural induction" (ERNI) promoter is used. An ERNI is a gene that is selectively expressed during the early stages of development (e.g. Stage X (E-G&K)) and in culture, and therefore, this promoter is used to drive expression of antibiotic resistance genes to select PGCs carrying a genetic modification. Since ERNI is only expressed during the early stages of development, the genes that confer antibiotic resistance are not expressed in the mature animals.

Example 11. Homogeneity of Long Term PGC Cell Cultures

To determine the homogeneity of PGC cultures after long-term culture, ES, EG, DT40 (chicken B cell line) and PGCs were stained with anti-CVH, an antibody against the chicken vasa homologue and the 1B3 antibody (Halfter, W., Schurer, B., Hasselhom, H. M., Christ, B., Gimpel, E., and Epperlein, H. H., An ovomucin-like protein on the surface of migrating primordial germ cells of the chick and rat. Development 122, 915-23. 1996)). Expression of the CVH antibody is restricted to germ cells and therefore, the anti-CVH antibody is a reliable marker for them. The 1B3 antigen recognizes an ovomucin-like protein present on the surface of chicken PGCs during their migration and colonization of the gonad.

Cells were washed in CMF/2% FBS, fixed in 4% paraformaldehyde for 5 minutes and washed again. The cell aliquots to be stained for vasa were permeabilized with 0.1 % TritonX-100 for 1-2 minutes. Primary antibody was added for 20 minutes, cells were washed twice and incubated with a secondary antibody (Alexa 488 anti-rabbit IgG for CVH and control and Alexa 488 anti-rabbit IgM for 1B3) for 15 minutes. As controls, aliquots of cells were stained only with second antibody. After an additional 2 washes the cells were prepared for FACS analysis.

Referring to Figure 9, DT40, ES and EG cells all show background when stained with CVH and the 1B3 Ab. PGCs , however, stain much stronger with both the CVH and the 1B3 antibody. There is a small population of PGCs, which do not stain for either CVH or 1B3 indicating that a small proportion of the cells do not display the PGC phenotype. Two parental PGC lines and 4 transfected cell lines (G-09, P84, P97/6 and P97/33) derived from the PGC13 parental cell line, were tested with the vasa and 1B3 antibody (PGC13 and 102). All show the same pattern indicating that the various PGC cultures contain the same high proportion of cells expressing the PGC phenotype.

Example 12: Genetic Modification of Primordial Germ Cells

Electroporation with a circular CX-GFP plasmid revealed that the rate of transient transfection in PGCs varied between 1-30%. Using 8 Square wave pulses of 100 μsec and 800V we obtained a PGC cell line carrying a CX-neo construct, that was designated G-09. See Figure 8. The integration of the construct was evaluated using Southern blot analysis. The isolation of this stably transfected line, however, was a spurious event that did not recur in subsequent experiments. With the exception of G-09, stable transfection of PGCs was not achieved after electroporating $17x10^7$ PGCs with linearized constructs in 37 transfection experiments using both square wave and exponential decay pulses. In each of these experiments, the number of PGCs varied from $1x10^6$ to $10x10^6$. The following promoters, used widely in ES cell research in mouse, chicken and human were tested: the CX promoter, also called CAG (Niwa, H., Yamamura, K., and Miyazaki, J., Efficient selection for high-expression transfectants with a novel eukaryotic vector. Gene 108, 193-9.1991)), which contains the chicken β-actin promoter with a CMV enhancer, the PGK promoter, the MC1 promoter and the Ubc promoter. None of these promoters increased transfection efficiency. To allow expression of selectable markers and clonal derivation of genetically modified cell lines, insulators have been used with integrated constructs.

Insulators are DNA sequences that separate active from inactive chromatin domains and insulate genes from the activating effects of nearby enhancers, or the silencing effects of nearby condensed chromatin. In chickens, the 5'HS4 insulator located 5' of the β-globin locus has been well characterized by Felsenfeld and colleagues (Burgess-Beusse, B., Farrell, C., Gaszner, M., Litt, M., Mutskov, V., Recillas-Targa, F., Simpson, M., West, A., and Felsenfeld, G. (2002)). The insulation of genes from external enhancers and silencing chromatin. Proc.. Natl. Acad. Sci. USA 99 Suppl. 4, 16433-7.. This insulator protects the β-globin locus from an upstream region of constitutively condensed chromatin. We assembled a transgene with the chicken β-actin promoter driving neomycin resistance using the chicken β-globin 5'HS4 sequence as insulators both 5' and 3' of the chicken β-actin-neo cassette.

The 250 bp core sequence of hypersensitive site 4 from the chicken β-globin locus was PCR amplified with the following primer set:

HS4-Bam-F: AGGATCCGAAGCAGGCTTTCCTGGAAGG (SEQ ID. NO. 7)

HS4-Bgl-R: AAGATCTTCAGCCTAAAGCTTTTTCCCCGT (SEQ ID. NO. 8)

The PCR product was cloned into pGEM-T and sequenced. A tandem duplication of the HS4 site was made by digesting the HS4 in the pGEM clone with BamHI and BglII to release the insert, and BglII to linearize the vector. The HS4 fragment was ligated to the vector containing a copy of the HS4 insulator. Clones were screened and one was selected in which the two copies of HS4 are in the same orientation. This is called 2X HS4.

Example 13: Bulk Selection Using HS4 β-Actin-neo.

β-actin neo was obtained from Buerstedde (clone 574) and transferred into pBluescript. 2X HS4 was then cloned at both the 5' and 3' ends of β-actin neo to produce HS4-β-actin neo. Eight transfections were performed using this construct. For each transfection $5x10^6$ PGCs were resuspended in 400 μl electroporation buffer (Specialty Media) and 20μg of linearized DNA was added. One Exponential Decay (ED) pulse (200V, with 900-1100μF) or eight Square Wave (SW) pulses (250-350V, 100μsec) were given. After transfection the cells were grown for several days before neomycin selection (300μg/ml) was added. Each transfection was grown as a pool. Resistant cells were isolated from 5 of 8 transfections

Southern analysis was performed on 2 pools of transfected cells (Figure 10). Two μg genomic DNA from PGC lines P84 and P85 and 20 pg of plasmid (HS4 -β-actin neo) were digested. Digests were run on a 0.7% gel, transferred by capillary transfer in 10X SSC to nylon membrane overnight, and probed with radiolabeled neo gene sequences for 2 hours in Rapid Hyb (Amersham). After washing, the blot was exposed to film overnight at -80°C. Referring to Figure 10, Lane1 is P84, Lane 2 is P85 and Lane 3 is the plasmid. For the plasmid control the HS4-β-actin-neo plasmid DNA was linearized with Not1. To obtain a 2.3 Kb internal fragment the PGC DNA and the linearized plasmid were digested with BamH1. Both P84 and P85 show an internal fragment of 2.3 Kb in size. A larger internal fragment of approximately 2.6 Kb was released by digestion with HindIII. Again this internal fragment is present in both the P84 and P85 digests. Digestion of genomic DNA of P84 and P85 with EcoR1 and BglII should reveal bands larger than 2.9Kb if the transgenes are integrated into the genome. In P84 no junction fragments are seen, indicating that P84 is a composite of several different clones. In P85, junction fragments of 4.5-5kb are present in the EcoR1 digestion and a junction fragment of 5Kb is present in the BglII digestion indicating that P85 is integrated into the genome and that the culture is comprised substantially from one clone. This example shows the utility of insulators as a preferred element of a construct for reliable expression of selectable markers in primordial germ cells.

Example 14: Clonal Derivation of Genetically Modified PGCs

The following examples show that genetically modified lines of primordial germ cells can be clonally derived.

First, β-actin-eGFP was made. The eGFP gene was released from CX-eGFP-CX-puro with XmnI and KpnI, β-actin was released from HS4-β-actin puro with EcoRI and XmnI, and the two were cloned as a 3-way ligation into pBluescript digested with EcoRI and KpnI to produce β-actin EGFP. Then, β-actin eGFP was released with BamHI and KpnI (blunted with T4 DNA polymerase) and cloned into HS4-β-actin puro digested with BglII and EcoRV.

Five transfections were performed using this construct. For each transfection $5x10^6$ PGCs were resuspended in 400μl electroporation buffer (Specialty Media) and 20μg of linearized DNA was added. An ED pulse (150-200V;900μF) or SW (350V, 8 pulses, 100μsec) pulses were given. After transfection the cells were plated into individual 48 wells and grown for several days before selection (0.5μg/ml) was added. A total of 5 clones were observed in 4 of the 5 transfections. One clone TP103 was analyzed by Southern (Figure 11). Referring to Figure 11, the plasmid control DNA was linearized with NotI. An internal fragment was released by digesting the DNA with KpnI. In both TP103 and the plasmid a fragment of the same size was released. Digestion of genomic DNA of TP103 with NcoI, MfeI, and SphI should reveal bands that are larger than the corresponding lanes of digested plasmid DNA. No band is seen in the lane of MfeI digested TP103 genomic DNA, which may be due to the band being too large. In the lanes representing the NcoI and SphI digestions, fragments have been released in the TP103 genomic DNA that are substantially larger than the fragments released in the plasmid DNA, indicating that the transgene is incorporated into the genome of the TP103 cell line.

*Clonal derivation of HS4-β-actin-puro.*

First, β-actin puro was made by a 3-way ligation of puro from CX-EGFP-CX-puro (XmnI-EcoRI), β-actin from β-actin neo in pBS (see above)(Sal-XmnI), and pBluescript (SalI-EcoRI). Next, β-actin puro was cloned into pBS containing two copies of 2X HS4 by ligating BamHI digested β-actin puro into BamHI/SAP treated 2X HS4 vector.

Three transfections were performed using this construct. For each transfection 4-5x$10^6$ PGCs were resuspended in 400μl electroporation buffer (Specialty Media) and 20μg of linearized DNA was added. An ED pulse was given of 200V, 900μF. After transfection the cells were plated into individual 48 wells and grown for several days before selection (0.5 μg/ml) was added. No colonies were seen in 2 transfections. Two colonies were isolated from the third transfection.

*Clonal derivation of HS4-cx-eGFP-cx-Puro.*

Three transfections were performed with HS4-cx-eGFP-cx-Puro. 5x$10^6$ PGCs were resuspended in 400μl electroporation buffer (Specialty Media) and 20μg of linearized DNA was added. Eight SW pulses of 350V for 100μsec was given to each transfection. After transfection the cells were plated in individual 48 wells, grown for several days before puromycin selection (0.5μg/ml) was added. A total of 16 clones were isolated from 2 transfections.

*Clonal derivation of cx-neo.*

The PGC 13 cell line was electroporated with a plasmid carrying a cx-neo selectable marker. After exposure to neomycin a cell line was derived that was resistant to neomycin (G-09). The karyotype of this cell line was determined and all cells exhibited a deletion in the p-arm of chromosome 2 (Table 3 and Figure 12). These data demonstrate that G-09 was clonally derived from a PGC carrying a signature deletion in the p-arm of chromosome 2.

Table 3: Chromosomal analysis of G-09 cell line.

| | Chromosomes | | | | | | |
|---|---|---|---|---|---|---|---|
| Cell | 1 | 2 | 2p- | 3 | 4 | Z | Micros |
| 1 | 2 | 1 | 1 | 2 | 2 | 2 | 69 |
| 2 | 2 | 1 | 1 | 2 | 2 | 2 | 44 |
| 3 | 2 | 1 | 1 | 2 | 2 | 2 | 56 |
| 4 | 2 | 1 | 1 | 2 | 2 | 2 | 56 |
| 5 | 2 | 1 | 1 | 2 | 2 | 2 | 65 |
| 6 | 2 | 1 | 1 | 2 | 2 | 2 | 67 |
| 7 | 2 | 1 | 1 | 2 | 2 | 2 | 59 |
| 8 | 1 | 0 | 1 | 1 | 2 | 1 | 38 |
| 9 | 2 | 1 | 1 | 2 | 2 | 2 | 65 |
| 10 | 2 | 0 | 1 | 2 | 2 | 2 | 55 |
| 11 | 2 | 1 | 1 | 2 | 2 | 2 | 43 |
| 12 | 2 | 1 | 1 | 2 | 2 | 2 | 59 |
| 13 | 2 | 0 | 1 | 2 | 2 | 2 | 55 |
| 14 | 2 | 0 | 1 | 2 | 2 | 2 | 33 |
| 15 | 1 | 1 | 1 | 2 | 2 | 2 | 56 |
| 16 | 2 | 1 | 1 | 2 | 2 | 2 | 62 |

Example 15: Tissue specific expression of selectable markers in PGCs.

The gene ERNI is expressed from the pre-primitive streak stage in the chicken embryo and is an early response gene to signals from Hensen's node Streit, A., Berliner, A. J., Papanayotou, C., Sirulnik, A., and Stem, C. D. (2000). Initiation of neural induction by FGF signalling before gastrulation. Nature 406, 74-8. Furthermore ERNI is expressed in chicken ES cells Acloque, H., Risson, V., Birot, A., Kunita, R., Pain, B., and Samarut, J. (2001). Identification of a new gene family specifically expressed in chicken embryonic stem cells and early embryo. Mech Dev 103, 79-91. The ERNI gene (also called cENS-1) has an unusual structure in which a single long open reading frame is flanked by a 486 bp direct repeat, in addition to unique 5' and 3' UTR sequences. Based on the idea that this structure is reminiscent of a retroviral LTR-like structure, Acloque et al. 2001 assayed different portions of the cDNA sequence for promoter/enhancer activity and found that a region of the unique sequence in the 3' UTR acts as a promoter. PCR primers were designed essentially as described (Acloque et al., 2001) to amplify an 822 bp fragment of the 3' UTR of the ERNI gene. After amplification of the ERNI sequences, they were cloned upstream of the neomycin-resistance gene, with an SV40 polyA site, to generate ERNI-neo (1.8 kb). The 2X HS4 insulator was then cloned on either side of the ERNI-neo selectable marker cassette.

Two transfections were performed with HS4-Erni-neo. $5x10^6$ PGCs were resuspended in 400μl electroporation buffer (Specialty Media) and 20μg of linearized DNA was added. In the first transfection a single ED pulse of 175V, 900μF was given and in the second transfecton, 8 SW pulses of 100μsec and 350V were given. After transfection the cells were plated in individual 48 wells, grown for several days before neomycin selection (300μg/ml) was added. In the first transfection (ED pulse) 5 colonies were isolated, and in the second transfection (SW pulses) 11 colonies were isolated.

The isolation of stably transfected clones indicates that ERNI is expressed in PGCs and can be used as a tissue specific promoter.

Example 16: Contribution of transfected PGCs to the germline.

PGCs were transfected with HS4-βactin-GFP and injected into the vasculature of Stage 13-15 (H&H) embryos. At D18, gonads were retrieved, fixed, sectioned and stained with the CVH antibody to identify the germ cells. The stained sections were then analyzed for the presence of GFP positive cells in the gonads. GFP positive germ cells were found in both male (Figure 13) and female gonads. Examination of histological preparations of brain, heart muscle and liver of these embryos showed only four green cells in one slide. These data demonstrate that a few cultured PGCs are found in ectopic locations but that the vast majority of cultured PGCs preferentially colonize the germline.

To determine that the GFP positive cells were germ cells the sections were stained with the anti-CVH antibody. As can be seen in Figure 14, the GFP positive cells also stain for the CVH protein, indicating that the GFP positive cells are germ cells.

Referring to Figure 14, GFP positive cells are present in this section and the DAPI/GFP panel shows that these GFP positive cells are located within the seminiferous tubule. When germ cells are stained with the anti-CVH antibody they exhibit a intense red stained ring that delineates the cytoplasm of the germ cells. The DAPI/CVH panel shows that these cells are located within the seminiferous tubule. The last panel shows that the GFP positive cells also stain for CVH and that the seminiferous tubules contains CVH positive germ cells that are GFP negative.

Example 17. Germline transmission of genetically modified PGCs.

Barred Rock PGCs transfected with one of the following transgenes: βactin-neo, βactin-eGFP-βactin-puro, cx-eGFP-cx-puro were injected into the vasculature of Stage 13-14 (H&H) embryos. The chicks were hatched, the roosters were grown to sexual maturity and bred to Barred Rock hens to determine germline transmission of the transgene. All black offspring were PGC derived and were tested for the presence of the transgene (Table 5). The rate of germline transmission was calculated by dividing the number of black chicks by the total number of chicks that were scored for feather color (Table 5).

Table 5: Germline transmission of genetically modified primordial germ cells.

| Cell line | Parental cell line | Age of cells | Construct | Roosters tested | # offspring | % gennline |
|---|---|---|---|---|---|---|
| TP84 | PGC 13 | 267 | βactin-neo | 5 | 892 | 0,0,0,0,0 |
| TP85 | PGC 13 | 260-267 | βactin-neo | 12 | 2462 | 0,0,0,0,0,0,0, 0,0,0,0.5, 1 |
| TP103/38 | PGC54 | 134-138 | βactin-GFP | 8 | 758 | 0,1,11,12,13,16,28,92 |
| TP112/44 | PGC 13 | 280 | cx-GFP | 4 | 168 | 0,0,0,4 |
| TP112/21 | PGC 13 | 280 | cx-GFP | 3 | 378 | 0, 1,10 |

Example 18. Transgenes are inherited in a Mendelian fashion

Black offspring from matings between chimeric roosters carrying Barred Rock PGCs that were genetically modified to include one of βactin-neo, βactin-GFP, or cx-GFP were analyzed for the presence of the transgene. As shown in Table 6 the transgene is inherited by approximately 50 % of the PGC offspring, indicating Mendelian inheritance.

Table 6. Mendelian segregation of the transgene.

| Construct | # black offspring | # non-transgenic offspring | # transgenic offspring |
|---|---|---|---|
| βactin-neo | 3 | 1 | 2* |
| βactin-GFP | 176 | 93 | 83* |
| cx-GFP | 23 | 9 | 14* |
| *Not significantly different from the expected 1:1 ratio of transgenic :non-transgenic offspring by Chi-square analysis | | | |

Example 19: Ubiquitous expression of transgenes in offspring of chimeras carrying genetically modified PGCs

Chimeras carrying PGCs in which βactin-GFP was stably integrated into the genome were mated with wild type hens and the embryos were scored for expression of GFP. Examples of expression in embryos are shown in Figure 15 which shows that GFP is expressed in all tissues of the transgenic offspring up to Stage 34 (H&H) of development. In older animals, tissues were prepared for histological examination using frozen sections. Tissues from pancreas, skin, lung, brain, ovary, kidney, bursa, duodenum. breast, heart, liver, and spleen of 1 to 2 week-old chicks demonstrated that expression remained ubiquitous in animals post hatching.

Example 20: Expression of a monoclonal antibody in egg white of transgenic chickens

As noted above, this monoclonal antibody is only one example of several types of monoclonal antibody products that may be expressed using the transgene constructs of the invention. Moreover, monoclonal antibodies as a class of proteins are only one example of many classes of protein products that may be expressed in tissue-specific fashion pursuant to the methods and techniques described herein. The following Example is used to express any protein or antibody with a known coding sequence.

The vector used to express monoclonal antibodies in the tubular gland cells of the chicken oviduct is designated OvBAC. This vector is comprised of an intact BAC clone from the chicken ovalbumin locus, including the ovalbumin structural gene and 5' and 3' flanking sequences. Insertion of a monoclonal antibody cassette (containing a gene encoding human IgL and a gene encoding human IgH, joined by an IRES sequence) into the ovalbumin gene on the BAC, such that the ovalbumin translation initiation codon is fused to the IgL initiator codon, drives expression of the monoclonal antibody in the oviduct to high levels. The antibody expressed in the oviduct is secreted and deposited into the egg white.

A monoclonal antibody modular cassette was designed in which unique restriction enzyme sites were placed strategically for the easy insertion of heavy and light chain variable region genes encoding the variable region of any desired monoclonal antibody leading to expression of full length human IgGκ. This cassette, once modified with the variable regions of interest, is then inserted into the OvBAC for expression of the MAb in the oviduct. The cassette contains the human Cκ constant region, the human Cγ1 constant region, and portions of the human kappa J-C intron and the intron upstream of human Cγ1. The signal peptide for the VH gene and a small intron downstream are also present; however, the VL signal peptide is not present. An IRES sequence is present between the IgL and IgH genes so that the complete antibody is expressed from a single transgene. Variable region genes are inserted into one of the unique restriction sites (such as *Sna*BI or *Srf*I for VL; *Nru*I or *Pme*I for VH) that are situated in the introns upstream of the constant region genes. The variable region genes must contain splice donor sequences so that they are spliced to the constant region genes for proper expression. Rearranged, expressed variable region genes are amplified by PCR from hybridoma genomic DNA or from recombinant DNA derived from the hybridoma. The signal peptide leader sequence for the light chain must be added at the time of amplification of the light chain V; the heavy chain signal peptide is present in the cassette and therefore is not needed when inserting the heavy chain V. For the VL gene, the PCR primers are designed to include the following. The 5' primer on the upstream side of the VL gene will include: a recognition site for the *Sna*BI restriction enzyme; the Kozak consensus ATG and signal peptide; and about 20 bp of homology to the V region of interest for priming in the PCR reaction. If a cDNA clone is used as the template in PCR, then the signal peptide exon will already be fused to the rest of the VL gene; otherwise, the primer will be designed to add a human VL signal peptide, in-frame, to the N-terminus of the mature variable region. This step may require two rounds of nested PCR to add the necessary sequences, since the primers will be long if the signal peptide is added in one step. On the 3' end of the VL gene, PCR primers are designed to include: a recognition site for the *Sgf*I restriction enzyme; about 20 bp of homology to the 3' end of the V region of interest; and about 20 bp of J-Cγ intron sequence, including the splice donor for splicing to the downstream Cγ gene. For the VH gene, the 5' primer for PCR amplification includes a recognition site for *Nru*I enzyme, about 20 bp of the VH intron that is present in the signal peptide sequence (including the splice acceptor for splicing to the VH signal peptide splice donor in the modular cassette), 11 bp of VH signal peptide coding sequence, and about 20 bp of homology to the 5' end of the VH gene of interest. The 3' primer includes about 20 bp of homology to the 3' end of the VH gene of interest (corresponding to the J region), about 20 bp of J-Cμ intron (including the splice donor for splicing to the Cγ1 gene downstream), and a recognition site for the *Nru*I enzyme. PCR products for VL and VH are cloned and sequenced before insertion into the modular MAb cassette vector.

The OvBAC clone is modified by recombineering to insert the MAb cassette into the ovalbumin sequences as described in Copeland, N. G., Jenkins, N. A., Court, D. L (2001). Recombineering: a powerful new tool for mouse functional genomics. Nat Rev Genet 2, 769-79. A selectable marker (for neo or puromycin resistance) is added to the OvBAC by retrofitting Wang, Z., Engler, P., Longacre, A., Storb, U. (2001). An efficient method for high-fidelity BAC/PAC retrofitting with a selectable marker for mammalian cell transfection. Genome Res. 11,137-42.

Example 21: Anti-IL-2Rα in egg white of transgenic chickens

The overall strategy to express MAb specific for the human IL-2Rα receptor is as follows (see Figures 16 and 18). In step 1, the anti- IL-2Rα IgL/IgH cassette is inserted into the Ov BAC by homologous recombination in E. coli by recombineering. The antibody is then under the transcriptional control of the Ov regulatory elements. In step 2, the kanamycin gene used in recombineering is removed by Flp recombinase. The V coding sequences from the humanized anti-IL-2Rα antibody are cloned into a cassette containing the Cκ and Cγ1 constant regions. The Igκ and IgH genes are joined by an IRES sequence, so that both genes are expressed from a single transgene construct and therefore only a single BAC transfection is needed. The antibody cassette is inserted into the Ov gene on the BAC by homologous recombination (Lee and Copeland, 2001). The Igκ gene is fused to the Ov Kozak translation initiation sequence for efficient translation. Finally, ERNI-puro which is a selectable marker in PGCs is added to the BAC for transfection and selection of PGC clones.

Figure 16 shows construction of OvBAC-anti-IL-2Rα. To obtain the heavy and light chain variable region genes, 4 long oligonucleotides (with about 20 bp overlap) for each gene are synthesized and annealed to each other. Gaps are filled in with DNA polymerase (from bacteriophage T4). The synthetic genes are then digested with restriction enzymes for cloning into the MAb cassette.

Oligonucleotides for the construction of the humanized anti-IL-2Rα Mab (MAb sequences are from patent 5,585,089) are as follows.

Light chain V gene (oligos 1-4):

Oligo 1: ctc TCTAGA caactcagagttcaccatg gagaccga taccctcctg ctatgggtcc tcctgctatg ggtcccagga tcaaccggag // atattcagat gacccagtct ccatctaccc tctctgctag cgtcggggat (SEQ ID. NO. 9)

Oligo 2: ataaattaga agcttgggag ctttgcctgg cttctgctgg taccagtgca tgtaacttat acttgagctg gcagagcagg ttatggtgac cctatccccg acgctagcag agag (SEQ ID. NO. 10)

Oligo 3: gctcccaagc ttctaattta taccacatcc aacctggctt ctggagtccc tgctcgcttc agtggcagtg gatctgggac cgagttcacc ctcacaatca gctctctgca gccagatgat ttc (SEQ ID. NO. 11)

Oligo 4: ctc GCGATCGC caatagtgaaaaattac gtttgac ctccaccttg gtcccctgac cgaacgtgag tgggtaagta ctcctttgat ggcagtaata agtggcgaaa tcatctggct gcagagagct ga (SEQ ID. NO. 12)

Referring to Figure 17, Oligo 1 has an XbaI site for cloning (capital letters), followed by the VL signal peptide (with no intron). The Ov Kozak translation initiation sequence is underlined; the last three nucleotides are the initiator codon. The signal peptide cleavage site (in the corresponding protein sequence) is indicated by a double slash. Oligo 4 has an SgfI site for cloning (capital letters), followed by 17 bp of the human 5' J4-Ck intron for splicing to Ck (underlined). The splice donor G nucletotide is double underlined.

Heavy chain V gene (oligos 5-8):

Oligo 5: ctc TCGCGA tctctgttcacag gcgtgcactct // cagg tccagcttgt ccagtctggg gctgaagtca agaaacctgg ctcgagcgtg aaggtc (SEQ ID. NO. 13)

Oligo 6: cccagtcgac ggattaatat atccaatcca ttccagaccc tgtccagggg cctgccttac ccagtgcatc ctgtagctag taaaggtgta gccagaagcc ttgcaggaga ccttcacgct cgagccagg (SEQ ID. NO. 14)

Oligo 7: tatattaatc cgtcgactgg gtatactgaa tacaatcaga agttcaagga caaggcaaca attactgcag acgaatccac caatacagcc tacatggaac tgagcagcct gagatctgag gaca (SEQ ID. NO. 15)

Oligo 8: ctc TCGCGA ggccattcttac ct gaggagactg tgaccagggt tccttggccc cagtagtcaa agaccccccc ccctcttgca cagtaataga ctgcggtgtc ctcagatctc aggctgct (SEQ ID. NO. 16)

Oligo 5 contains an NruI site (capital letters) for cloning, followed by 15 bp of the 3' end of the human VH signal peptide intron (underlined), followed by 11 bp of the VH signal peptide exon sequence from the humanized anti-IL-R2α VH gene (double underlined). The signal peptide cleavage site (in the corresponding protein sequence) is indicated with a double slash. Oligo 8 contains an NruI site (capital letters) followed by 12 bp of the 5' end of the human J-Cμ intron (underlined). The splice donor C nucleotide is double underlined.

Oligos 1-4 are mixed, oligos 5-8 are mixed, and the two mixtures are annealed by incubating in a beaker of boiling water which is allowed to cool slowly to room temperature. Gaps in the complementary strands are repaired with DNA polymerase.

Referring to Figure 18, the Igκ and IgH Vs are then cloned into the cassette containing the Cκ and Cγ1 genes, using unique restriction sites designed into the 5' and 3' ends of the Vs (in this example, NruI for the heavy chain V and XbaI/SgfI for the light chain V).

Referring again to Figure 18, the OvBAC is shown on the top, with 110 kb of sequence 5' of the Ov structural gene, and 30 kb of flanking sequence 3' of the Ov structural gene. The ERNI-puro selectable marker is shown at the 3' end. The MAb cassette is shown with the following elements (left to right): the 5' Ov homology arm for insertion into the OvBAC by homologous recombination; the Ov Kozak and ATG; the human VL signal peptide (SiGVL); the inserted human light chain variable region gene from the MAb (VL); the J-Cκ intron; the Cκ gene; the IRES for translation of the downstream IgH gene; the human heavy chain signal peptide (SiGVH); the inserted heavy chain variable region gene from the MAb (VH); the J-Cγ intron; the Cγ1 gene including its internal introns; and the 3' Ov homology arm for insertion into the OvBAC. (The Kanamycin gene for selection in bacteria is not shown.)

For insertion of the antibody cassette into the OvBAC, a recombineering targeting vector is made by adding homology arms to the Igκ-IRES-IgH cassette. The homology arms are fragments from the Ov locus that act to target the antibody cassette to the Ov gene in the BAC, using the homologous recombination machinery in the EL250 *E. coli* strain harboring the BAC (Lee and Copeland, 2001). The 5' Ov homology arm is 124 bp of ovalbumin sequence corresponding to a HincII - XbaI fragment located immediately upstream of the Ov Kozalc translation initiation sequence in the Ov gene and has the following sequence:

5'-gttaacatttaattgcctaaaaactgctcgtaatttactgttgtagcctaccatagagtaccctgcatggtactatgtac agcattccatccttacattttcactgttctgctgtttgctctaga-3' (SEQ ID. NO. 17)

The 5' homology is PCR amplified from chicken genomic DNA or cloned Ov DNA using the following primers:

K8 HincII-F 5'- GGA TAT AGC AAC AGA CAC ATT AC-3' (SEQ ID. NO. 18)

K8-TTT NotIXbaI-R 5'-TTT GCG GCC GCT CTA GAG CAA ACA GCA GAA C-3' (SEQ ID. NO. 19)

The 3' Ov homology arm is 125 bp of ovalbumin sequence located immediately downstream of the translation termination codon of ovalbumin and has the following sequence:

5'-aaagaagaaagctgaaaaactctgtcccttccaacaagacccagagcactgtagtatcaggggtaaaatgaaaagtatgt tatctgctgcatccagacttcataaaagctggagcttaatctaga-3' (SEQ ID. NO. 20)

The 3' Ov homology is obtained as a 152 bp PCR product amplified from chicken genomic DNA or cloned Ov DNA using the following primers:

NotI OV (3'TAA)-F 5'-AAAAGCGGCCGCAAAGAAGAAAGCTGAAAAAC-3' (SEQ ID. NO. 21)

3'OVTAA-R2 5'-CTCCGCGGCTCGAGTCTAGATTAAGCTCCAGCTT-3' (SEQ ID. NO. 22)

Following amplification of the 5' and 3' homology fragments, the PCR products are cloned into a plasmid vector such as pBluescript and confirmed by sequencing. Then the homology arms are placed on either side of the MAb cassette; the 5' Ov homology is placed on the 5' side of the IgL gene and the 3' Ov homology is placed 3' of the IgH gene. Insertion of the MAb cassette into the OvBAC by homologous recombination results in the deletion of the Ov structural gene. The final structure of the MAb cassette for targeting into the Ov BAC is also shown in Figure 17.

A selectable marker, such as a gene encoding kanamycin resistance, is required for selection of homologous recombinants in the E. coli harboring the OvBAC following transformation with the MAb cassette. Thus, the targeting vector also contains a kanamycin-resistance gene flanked by FRT sites. For example, a 1.5 Kb FRT-Kan cassette is released from pIGCN21 (a vector containing the IRES-eGFPcre-FRT-kan-FRT cassette, obtained from Neal Copeland's lab at NCI) by Xma I and Bgl I (Nt4644-6131) and blunted. This fragment is then inserted into the blunted Not I site in the MAb cassette flanked by Ov homology. The targeting vector is electroporated into bacteria carrying the wild type OvBAC and kanamycin-resistant colonies are selected. Correct targeting is assessed by restriction mapping of the clones. Most of the kanamycin-resistant colonies should be correctly targeted. The resistance cassette is then removed by transient expression of Flp recombinase by arabinose induction of the Flp gene in the EL250 strain, resulting in OvBAC-anti-IL-2Rα. Kanamycin sensitive clones are screened and verified by restriction mapping.

For selection of BAC-transformed PGC cells, a selectable marker active in PGCs is then added to the BAC. We have used the puromycin resistance gene driven by the ERNI promoter to derive stably transformed PGC lines. ERNI is a gene expressed specifically in early chicken embryos, so the ERNI-puro marker will not be expressed in adult transgenic chickens. We have also found that flanking the selectable marker with the insulator element from the chicken β-globin locus increased the number of PGC colonies obtained after transfection. This element, called HS4, is thus cloned on either side of ERNI-puro. HS4-ERNI-puro is added to the BAC by retrofitting (Wang et al., 2001). The final OvMAb anti-IL-2Rα BAC is linearized with AscI before transfection.

Example 22: Chemical Properties of Antibodies Produced in Egg White of Transgenic Chickens.

The chemical properties of antibodies produced in the tubular gland cells of transgenic chickens will exhibit unique properties. U.S. patent application Serial No. 11/049,229 and (Zhu, L., et al. Nat. Biotech. 23: 1159-1169 2005) are specifically incorporated herein by reference. Specifically, monosaccharide analysis of antibodies produced in chimeric chickens reveal a difference in carbohydrate composition and show the presence of N-acetyl glucosamine residues, mannose residues, and very low content of galactose residues. Transgenic chickens will exhibit the same properties.

The most differences in the N-linked oligosaccharide profiles are the presence of high mannose type N-glycans, the absence of fucose and the very low content of galactose residues in the antibody produced in the chicken. These properties are important for several reasons. Firstly, there is no evidence of a $\alpha$1-3 Gal linkages, which are known to be antigenic. The reduction in galactose concentrations, typically to levels less than approximately 2%, substantially reduces antigenicity resulting from the galactose-containing linkages. Secondly, there is no evidence for N-glycolylneuraminic acid residues, which are also known to be antigenic. Thirdly, the antibody produced in chicken tubular gland cells are substantially free of fucosyl residues, which enhances the ADCC activity of antibodies. In this context, substantially free is defined as less than 0.1 %. Fourth, the chicken produced antibody has a high mannose content, typically greater than 40%, which increases the rate of clearance of this antibody when clearance was assessed in Balb/c mice using antibody produced in a CHO cell as the standard. Together with these advantageous chemical properties, the antibodies are expected to be present in egg white at concentrations not observed with transgenes that are randomly integrated into the chicken genome or which are not expressed in a tissue specific manner. Preferred concentrations are greater than one mg of antibody per egg, greater than 2 mg per egg, greater than 3 mgs per egg, and as high as 6 mgs per egg. Because each egg comprises approximately 25 ml of egg white, preferred concentrations are greater than 40 $\mu$g/ml, greater than 80 $\mu$g/ml, greater than 120 $\mu$g/ml, and as high as 240 $\mu$g/ml.

To extract and purify antibody from egg white, egg white is first mixed at a low shear rate for 30 min at room temperature and then ovomucin precipitated by a modified method described previously. One volume of homogenized egg white suspension is added to three volumes of reverse osmosis water and stirred for 30 min. The diluted suspension is adjusted to pH 6.0 using 0.5 M phosphoric acid and then centrifuged for 20 min at 12,100g. The supernatant is adjusted to pH 7.4 using 0.5 M dibasic sodium phosphate and 150 mM sodium chloride concentration with crystalline salt. The human IgG is purified on a Protein A-Sepharose FF column (Amersham Biosciences) at a 120 cm/h linear flow rate. The adsorbed human IgG is washed with five column volumes of the loading buffer (PBS, pH 7.4) and then eluted with 3 mM phosphoric acid. The eluted human IgG fraction is adjusted to pH 7.5 using 60 mM sodium phosphate (pH 7.5) containing 230 mM NaCl to achieve a final concentration of 40 mM sodium phosphate and 150 mM NaCl. The sample was then filtered through a 0.2 mm syringe filter (Pall).

Example 23: Assay for binding affinity

PSMA on LNCaP cells (ATCC) was used as antigen to assay for binding. Two hundred thousand cells/well were incubated in duplicate for 30 minutes with 50 $\mu$l aliquots of antibody at the indicated concentrations. Cells were washed twice before addition of goat anti-human IgG PE labeled antibody (Jackson ImmunoResearch) at 1:200 dilution, 50 $\mu$l /well for 30 minutes at 4°C. Cells were washed twice in PBS with 1% BSA and assayed by FACS. $EC_{50}$ values of MAb binding to PSMA on LNCaP cells were determined from binding curves utilizing GraphPad Prism 3.0 (GraphPad Software). Cells were grown in RPMI 1640 medium supplemented with 10% FBS, 10 mM HEPES, 2 mM L-glutamine, and 1 mM sodium pyruvate. The antigen binding property of MAbF1 produced in chicken tubular gland cells was compared with that of MAbF1 produced in CHO cells. Both antibody preparations produced nearly identical binding curves to PSMA expressed on LNCaP cells with similar $EC_{50}$ values. The data demonstrate that while the chicken-derived and CHO-derived antibodies are glycosylated differently, they recognize and bind antigen equivalently.

Example 24: Antibody internalization assay

Binding of the MAbF1 to PSMA leads to internalization of the antibody. In one potential application, MAb could be conjugated with cytotoxins in order to target and destroy PSMA-expressing tumor cells. Internalization of antibody binding to PSMA on LNCaP cells was determined by incubating cells, with MAb and Hum-Zap (Advanced Targeting Systems). HumZap is a goat anti-human IgG antibody conjugated to the ribosome inactivating protein, saporin. Cells are killed when the MAbF1/Hum-Zap complex binds to PSMA on the cell surface and is internalized whereas antibody or Hum-Zap alone is not toxic to LNCaP cells. LNCaP cells (10,000 / well) were incubated in triplicate, for 48 hours, at 37°C, in 150 $\mu$l of culture medium containing 300 ng Hum-Zap, and 300 ng of F1 MAb, or control MAb. Cell proliferation and survival was determined with the CellTiter-Glo Luminescent Cell Viability Assay (Promega). Internalization assays were also done by incubating dilutions of antibody in cell culture medium with 10,000 adherent LNCaP cells/well, for 2 hours at 4°C. Antibody solutions were gently removed and replaced with 150 $\mu$l of medium containing 200 ng of HumZap. Cell viability was determined following 48 hours of incubation at 37°C. $EC_{50}$ values for antibody internalization were determined graphically with Prism 3.0 (GraphPad Software). Both antibody preparations internalize with a similar efficiency. When tested over a range of antibody concentrations, the $EC_{50}$ values for internalization of both the chicken-derived and CHO-derived MAbF1 were 0.49 nM.

Example 25: Clearance of MAb in BALB/c mice

The *in vivo* half-life of the chicken produced MAbF1 was analyzed in parallel with the CHO produced antibody in BALB/c mice by intravenous injection of radiolabeled antibodies. Ten $\mu$g of MAb protein were lightly iodinated (less than one I per antibody) with $^{125}I$ using the Iodobead method (Pierce). Six week-old female BALB/c mice (Taconic Farms, Germantown, NY) were fed 0.1 mg/ml potassium iodide in their drinking water for one week prior to the experiment. Four mice per protein were injected intravenously into the tail vein with approximately 600,000 cpm of labeled MAb and whole body radioactivity was measured at selected times using a whole body gamma counter (Wm. B. Johnson NaI crystal detector with a Ludlum scaler). Half-life was calculated by exponential regression analysis of the residual radioactivity. MAbF1 produced by chicken tubular gland cells cleared with a half-life ($t_{1/2}$) of 102.4± 0.9 hours, while MAbF1 produced by CHO cells cleared more slowly with a half-life of 207.5±18.3 hours.

Example 26: Assay for ADCC

LNCaP-C42B cells were tested in a modified $^{51}Cr$ ADCC assay. Human peripheral blood mononuclear cells were purified from heparinized whole blood by standard Ficoll-paque separation. The cells were resuspended (at 1x10E6 cells / mL) in RPMI1640 media containing 10% FBS and 10 U/ml of human IL-2 and incubated overnight at 37°C. The following day, the cells were collected and washed once in culture media and resuspended at 2 x $10^7$ cells /ml. Two million target LNCaP-C42b cells are incubated with 200 uCi $^{51}Cr$ in 1 ml total volume for 1 hour at 37° C. The target cells are washed once, resuspended in 1ml of media, and incubated at 37° C for an additional 30 minutes. After the final incubation, the target cells are washed once and brought to a final volume of 1x$10^5$ cells/ml. For the final ADCC assay, 100 $\mu$l of labeled LNCaP cells are incubated with 50 $\mu$l of effector cells and 50 $\mu$l of antibody. The final target to effector ration of 1.100 was selected. In all studies, human IgG1 isotype control is run and compared to CHO-derived anti-PSMA MAbF1 antibody. Other controls which are included are: a) target and effector cells but no antibody, b) target cells with no effector cells and c) target and effector cells in the presence of 3% Triton X-100. Following 4 hour incubation at 37° C, the supernatants were collected and counted on a gamma Counter (Cobra II auto-gamma from Packard Instruments) with a reading window of 240-400 keV. The counts per minute were plotted as a function of antibody concentration and the data was analyzed by non-linear regression, sigmoidal dose response (variable slope) using Prism software (San Diego, CA). The percent lysis was determined by the following equation:

$$\% \text{ Lysis} = (\text{Sample CPM- No antibody CPM})/\text{TritonX CPM-No antibody CPM}) \text{ X } 100$$

Both $EC_{50}$ values and % Lysis are monitored in all studies. For example, it is possible when comparing two antibodies to have a change in either the $EC_{50}$ or % lysis or both.

Blockade of ADCC with anti-CD16 antibodies was conducted with the following modifications. The cells were incubated with either 1 or 0.01 μg/ml of CHO produced or chicken produced MAbF1 antibodies in the absence or presence of 5 μg/ml of anti-CD 16 antibody 3G8 or isotype control antibody.

CHO-derived MAb induce dose dependent cell lysis which reaches a plateau at 38% lysis with an $EC_{50}$ of 0.11 μg/ml with IL-2 stimulated effector cells. In contrast, the chicken egg derived MAb was more potent and more efficatious. The maximum % lysis of the chicken egg derived MAb was 60% with two different preparations of the antibody. The enhanced potency over the CHO derived MAb was also demonstrated as the $EC_{50}$ of this material was 0.018 μg/ml. Finally, as expected, isotype control antibody did not induce cell lysis. ADCC with unstimulated effector cells (fresh PBMCs) shows a greater difference in EC50 values, but lower overall cell killing.

CD16 (FCgRIII) is a key receptor that mediates ADCC. The specificity of the ADCC response was shown by blocking the interaction of target and effector cells using a monoclonal antibody directed against CD16. In this study, two doses of MAbF1 antibody were used, a saturating dose (1 μg/ml) and a sub-optimal dose (0.01 μg/ml). One μg/ml of MAbF1 antibody, in the absence of anti-CD16 antibody, induced approximately 15% and 38% lysis with CHO-derived and chicken-derived antibody, respectively. This % lysis was reduced to ~4% in the presence of anti-CD16 antibody while isotype control antibody had no effect.

Example 27: CD16 binding

CHO and Chicken derived MAbF1s were immobilized to a carboxymethyl dextran matrix surface of a Biacore sensor chip (CM5) via primary amines, using amine coupling kit provided by Biacore. Both antibodies were coated to a density of about 10,000 RUs. The binding of the two antibodies with CD16-Phe and CD16-Val were carried out by flowing several concentrations of the proteins over the immobilized antibody surfaces. Non-specific binding effects were accounted for by considering blank surfaces and plain buffer binding cycles. HBS-EP buffer was used for dilutions and as running buffer. The experiment was conducted at 25 °C on a Biacore-3000 instrument. Data was analyzed using GraphPad Prism software and data was fitted to a single binding site model to estimate the equilibrium dissociation constant.

The dissociation constant was estimated based on equilibrium binding experiments rather than rate constants, since fast kinetics is a characteristic feature of FcRs binding to antibodies. The dissociation constant, $K_D$, of chicken derived antibody is about ten fold lower for both the FcRs, compared to the corresponding CHO derived antibody. The higher affinity of the chicken derived antibody may be attributable to the differences in the glycosylation in the Fc region, especially due to the absence of fucose, which is present in the CHO derived antibody.

Example 28: Therapeutic utility

The present invention provides antibodies having specifically defined glycosylation patterns and other chemical properties and which have been generated using the genetically modified chicken described above. These properties provide improved therapeutic properties when administered to a patient for the purpose of binding to antigen-specific targets in target tissue. Specifically, as noted above, for certain clinical indications, the antibodies exhibit enhanced antibody-dependent cellular cytotoxicity (ADCC) and this effect offers important advantages in certain clinical indications.

Clinical trials of unconjugated monoclonal antibodies (mAbs) for the treatment of some types of cancer have yielded encouraging results. Dillman, 1997, Cancer Biother. & Radiopharm. 12:223-225; Deo et al., 1997, Immunology Today 18:127. A chimeric, unconjugated IgG1 has been approved for low-grade or follicular B-cell non-Hodgkin's lymphoma (Dillman, 1997, supra), while another unconjugated mAb, a humanized IgG1 targeting solid breast tumors, has also shown promising results in phase III clinical trials. Deo et al., 1997, supra. The antigens of these two MAbs are highly expressed in their respective target tissue. For such applications, particularly in tumor cells where the antibodies mediate potent tumor desctruction by ADCC, the antibodies of the present invention offer therapeutic advantages upon administration to a patient.

For therapeutic uses, an antibody of the invention can also be functionally linked (e.g., by chemical coupling, genetic fusion, noncovalent association or otherwise) to one or more other molecular entities, such as another antibody (e.g., to produce a bispecific or a multispecific antibody), a cytotoxin, cellular ligand or antigen (e.g., to produce an immunoconjugate, such as an immunotoxin). An antibody of the present invention can be linked to other therapeutic moieties, e.g., a radioisotope, a small molecule anti-cancer drug, an anti-inflammatory agent, a cytotoxin or an immunosuppressive agent. Accordingly, the present invention encompasses antibody compositions having chemical properties enabled by the chicken expression system and combined with essentially all known antibody conjugation, linking, and related technology for therapeutic use.

Accordingly, antibodies of the present invention can be used to treat and/or prevent a variety of diseases involving cells expressing antigen in a target tissue that is susceptible to treatment, particularly when the ADCC mechanism is exhibited in target tissue. Exemplary diseases that can be treated (e.g., ameliorated) or prevented include, but are not limited to solid tumors, lymphomas, diffuse tumors, and cancerous tissues of all types.

In a therapeutic embodiment of the invention, a patient is administered the antibody of the invention specifically in accord with a diagnosis of a condition that would be treated by a modality exhibiting the property of ADCC. In such a clinical setting, the antibodies of the invention are administered and the cellular cytotoxic effect of the treatment is determined following the treatment to determine the ADCC effect in target tissue. In addition to the therapeutic compositions of the invention, the patient may be additionally treated with a chemotherapeutic agent, radiation, or an agent that modulates, e.g., enhances or inhibits, the expression or activity of an Fc receptor, such as a cytokine. Typical, cytokines for administration during treatment include granulocyte colony-stimulating factor (G-CSF), granulocyte-macrophage colony-stimulating factor (GM-CSF), interferon-$\gamma$ (IFN-$\gamma$), and tumor necrosis factor (TNF). Typical therapeutic agents include, among others, anti-neoplastic agents such as doxorubicin, cisplatin, bleomycin, cannustine, chlorambucil, and cyclophosphamide.

In another aspect, the present invention provides a composition, e.g., a pharmaceutical composition, containing one or a combination of the chicken-expressed antibodies of the present invention. A composition of the present invention can be administered by a variety of methods known in the art. As will be appreciated by the skilled artisan, the route and/or mode of administration will vary depending upon the desired results. Pharmaceutically acceptable carriers include sterile aqueous solutions or dispersions and the use of such media and agents for pharmaceutically active substances is known in the art. Sterile injectable solutions can be prepared by incorporating the active compound in the required amount in an appropriate solvent with one or a combination of ingredients followed by sterilization and/or microfiltration. Generally, dispersions are prepared by incorporating the active compound into a sterile vehicle that contains a basic dispersion medium and any other ingredients.

Dosage regimens are adjusted to provide the optimum desired ADCC effect. For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

Because the effect of the administration of antibodies of the invention is objectively observable in target tissue such as tumors, the therapeutic methods of the invention include diagnosing a patient in need of therapy, including specifically a therapy using ADCC, identifying target tissue in which the effect is desired, administering the compositions of the invention to the patient in need thereof, and measuring the therapeutic effect in the patient, such as by determining the efficacy of the ADCC in the target tissue of the patient. The determination of therapeutic effect may be achieved by analyzing changes in the properties of the target tissue over time, such as cell death, shrinkage of target tissue, reductions in tumor size, and any other diagnostic technique known in the medical arts.

The antibodies of the invention can also be tested for ADCC activity in any of a number of known models for ADCC available to those skilled in the art. For purposes of determining the utility of the present antibodies for therapeutic or diagnostic use, the measurement of ADCC may be performed independently or compared with other mammalian, non-mammalian, plant, or bacterial cell expression systems. Accordingly, the methods of the invention include determining the difference in utility for purposes of effecting ADCC by using an antibody of the present invention in direct or indirect comparison to another antibody produced in the aforementioned systems. Specifically, this methodology includes comparing the ADCC effect of antibodies produced in the chicken expression system described above to identify enhanced ADCC to identify ideal antibody candidates for the chicken expression system.

As noted above, to enhance the therapeutic utility, the antibodies of the invention can be co-administered with one or other more therapeutic agents, e.g., a cytotoxic agent, a radiotoxic agent or an immunosuppressive agent. The antibody can be linked to the agent (as an immunocomplex) or can be administered separate from the agent. In the latter case (separate administration), the antibody can be administered before, after or concurrently with the agent or can be co-administered with other known therapies, e.g., an anti-cancer therapy, e.g., radiation. Such therapeutic agents include, among others, anti-neoplastic agents such as doxorubicin, cisplatin, bleomycin, carmustine, chlorambucil, and cyclophosphamide. Coadministration of the antibodies of the present invention with chemotherapeutic agents provides two anti-cancer agents which operate via different mechanisms which yield a cytotoxic effect to human tumor cells. Such co-administration can solve problems due to development of resistance to drugs or a change in the antigenicity of the tumor cells which would render them unreactive with the antibody.

Some non-limiting features of the invention are as follows:

A. A transgenic chicken comprising:

germline tissues colonized by genetically modified primordial genn cells comprising a transgene encoding exogenous DNA of a size greater than 15 kb.

B. The transgenic chicken of clause A wherein the transgene is stably integrated.

C. The transgenic chicken of clause B wherein the exogenous DNA encodes a protein.

D. The transgenic chicken of clause C wherein protein is a monoclonal antibody.

E. The transgenic chicken of clause A wherein the transgene is comprised of a human polynucleotide sequence.

F. The transgenic chicken of clause A wherein the exogenous DNA is flanked by at least one insulator.

G. The transgenic chicken of clause A wherein the transgene is comprised of a selectable marker.

H. The transgenic chicken of clause A wherein the transgene is comprised of the ERNI promoter.

I. A transgenic chicken whose genome is comprised of a transgene comprised of exogenous DNA greater than 10 kb.

J. The transgenic chicken of clause I wherein the transgene is stably integrated.

K. The transgenic chicken of clause I wherein the exogenous DNA encodes a protein.

L. The transgenic chicken of clause I wherein protein is a monoclonal antibody.

M. The transgenic chicken of clause I wherein the transgene is comprised of a human polynucleotide sequence.

N. The transgenic chicken of clause I wherein the exogenous DNA is flanked by at least one insulator.

O. The transgenic chicken of clause A wherein the transgene is comprised of a selectable marker.

P. The transgenic chicken of clause A wherein the transgene is comprised of the ERNI promoter.

Q. A method to produce a transgenic chicken comprising:

incorporating a transgtene greater than 10 kb into a primordial germ cell,
inserting the primordial geml cell into a recipient embryo,
hatching a transgenic chicken.

R. The method of clause Q wherein the transgene is stably integrated.

S. The method of clause Q wherein the exogenous DNA encodes a protein.

T. The method of clause Q wherein protein is a monoclonal antibody.

U. The method of clause Q wherein the transgene is comprised of a human polynucleotide sequence.

V. The method of clause Q wherein the exogenous DNA is flanked by at least one insulator.

W. The method of clause Q wherein the transgene is comprised of a selectable marker.

X. The method of clause Q wherein the transgene is comprised of the ERNI promoter.

Y. A clonally-derived cell culture of chicken primordial germ cells carrying exogenous DNA stably integrated into the genome of the primordial germ cells.

Z. The culture of clause Y5 wherein the culture medium is comprised of conditioned media from buffalo rat liver (BRL) cells.

AA. The culture of clause Z wherein the culture medium is comprised of :fibroblast growth factor (FGF).

AB. The culture of clause Z wherein the culture medium is comprised of stem cell factor.

AC. The culture of clause Z wherein the culture medium is comprised of chicken serum.

AD. The culture of clause Z wherein the culture comprises at least 1 X $10^5$ cells.

AE. The culture of clause Z wherein the exogenous DNA is flanked by at least one insulator.

AF. A germline chimeric chicken comprising:

gemlline tissues colonized by genetically modified primordial gelm cells, and

somatic tissues substantially free of genetically modified cells.

AG. The germline chimera of clause AF wherein the genetic modification is the stable integration of exogenous DNA.

AH. The germline chimera of clause AG wherein the exogenous DNA encodes a protein.

AI. The germline chimera of clause AF wherein protein is a monoclonal antibody.

AJ. The germline chimera of clause AH wherein the monoclonal antibody has a human polynucleotide sequence.

AK. The germline chimera of clause AH wherein the exogenous DNA is flanked by at least one insulator.

AL. A primordial germ cell comprising a stably integrated transgene having an expressable early response to neural induction (ERNI) promoter operatively linked to a coding region of the transgene.

## Claims

1. A method to produce a transgenic chick comprising:

   incorporating a transgene flanked by at least one insulator into the genome of a primordial germ cell, obtained by culturing whole blood of a stage 12 to 17 embryo in media conditioned with buffalo rat liver (BRL) cells and containing fibroblast growth factor (FGF), stem cell factor, chicken serum and non-chicken feeder cells, inserting the primordial germ cell into a recipient embryo to produce a germline chimera having the trans gene in its germline, raising the germline chimera to sexual maturity, and
   breeding the germline chimera to obtain a transgenic chicken having the trans gene stably integrated in its genome.

2. The method of Claim 1, wherein the transgene comprises exogenous DNA of a size greater than 10 kb, or greater than 15 kb.

3. The method of Claim 1, wherein the transgene comprises exogenous DNA encoding a protein.

4. The method of Claim 3, wherein the protein is a monoclonal antibody.

5. The method of Claim 1, wherein the transgene comprises a human polynucleotide sequence.

6. The method of Claim 1, wherein the at least one insulator is a HS4 insulator.

7. The method of Claim 1, wherein the transgene comprises a selectable marker.

8. The method of Claim 1, wherein the transgene comprises the ERNI promoter.

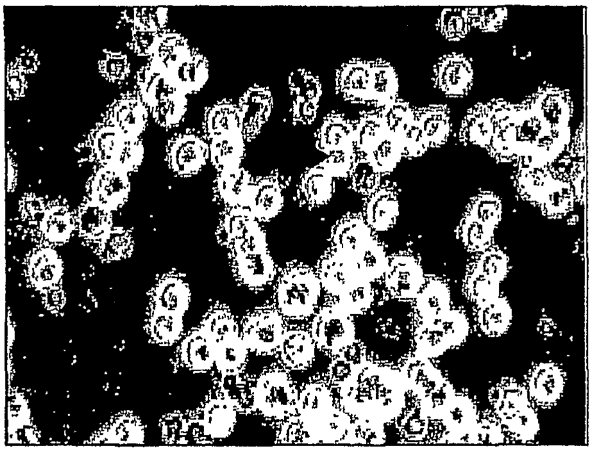

FIGURE 1A

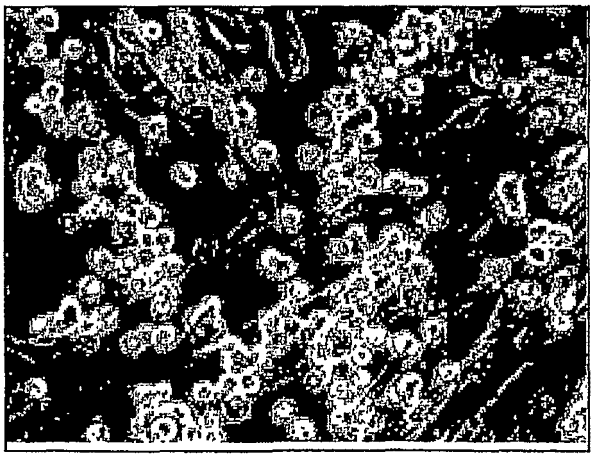

FIGURE 1B

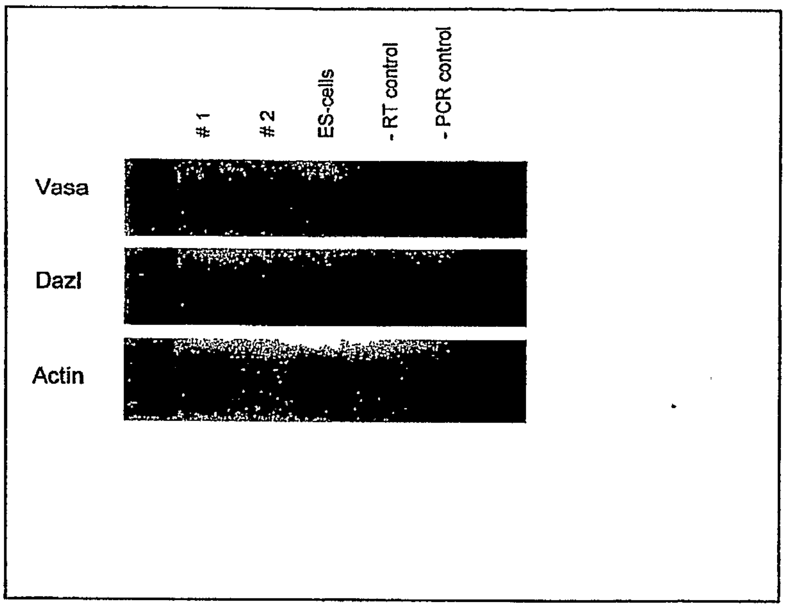


FIGURE 2

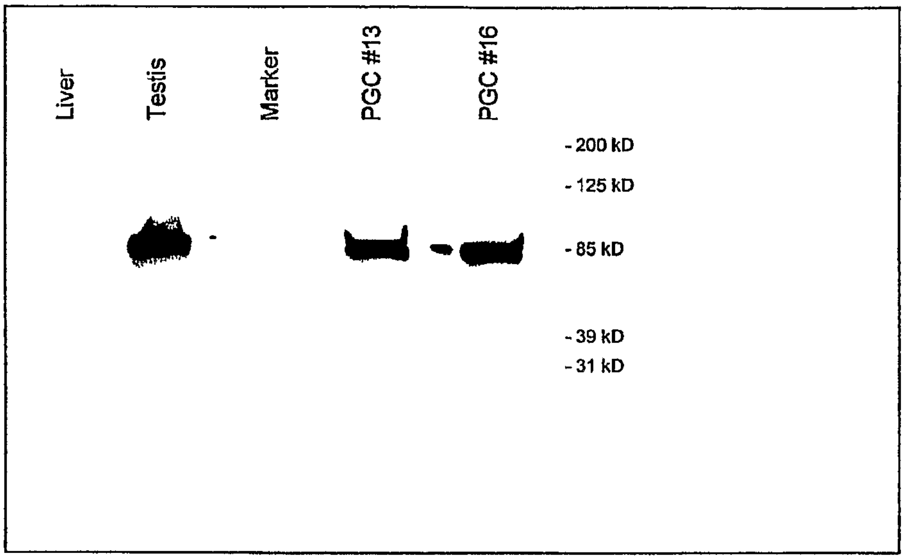


FIGURE 3

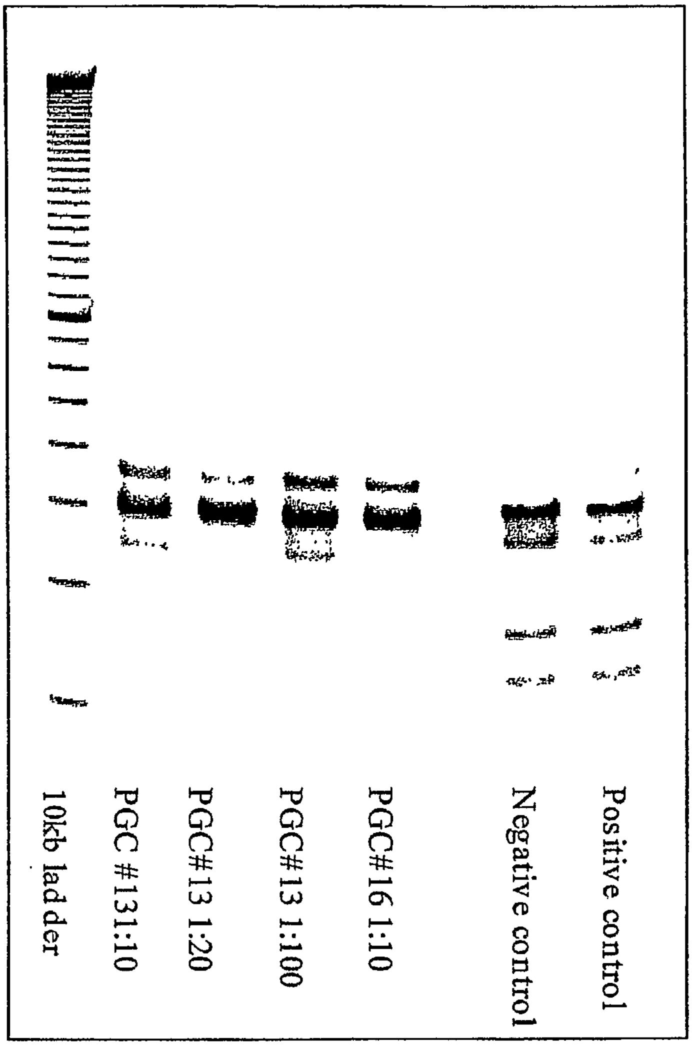
10kb ladder
PGC #13 1:10
PGC#13 1:20
PGC#13 1:100
PGC#16 1:10
Negative control
Positive control

FIGURE 4

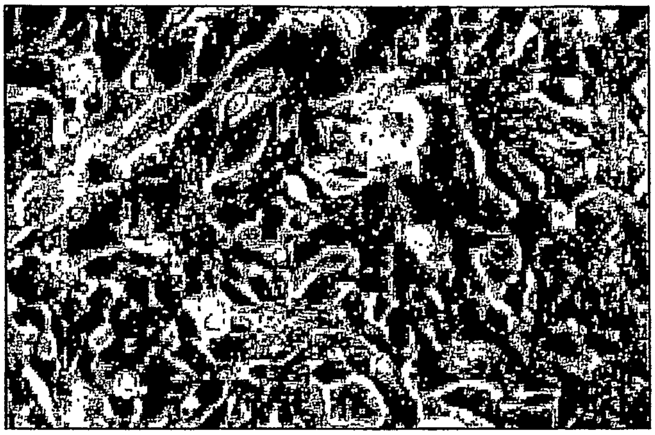

FIGURE 5A

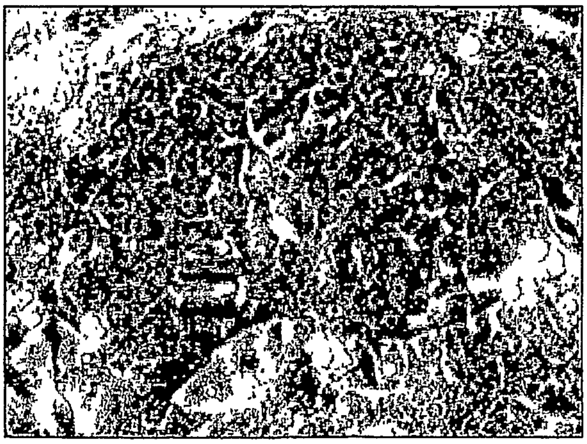

FIGURE 5B

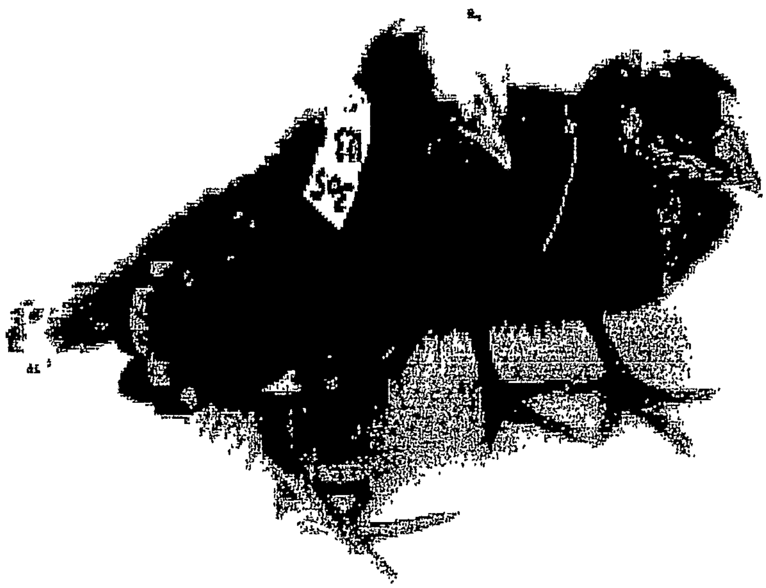

FIGURE 6

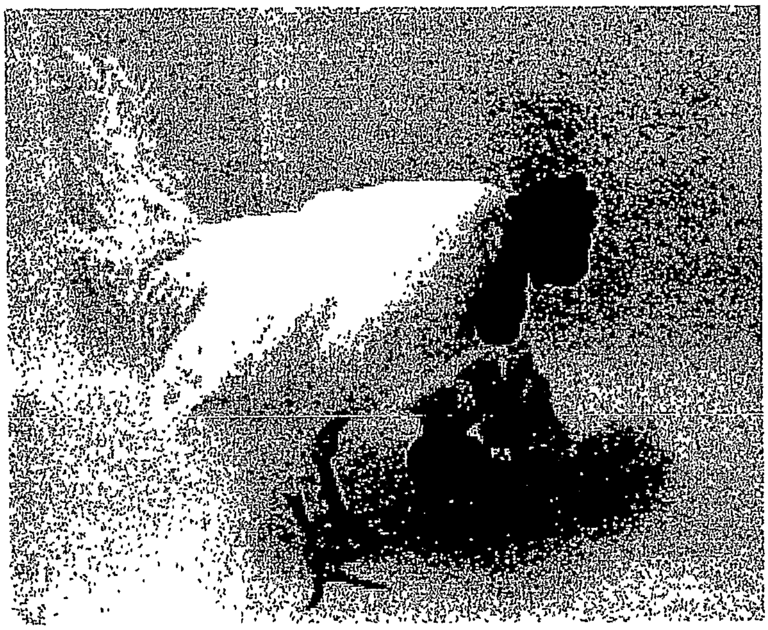

FIGURE 7

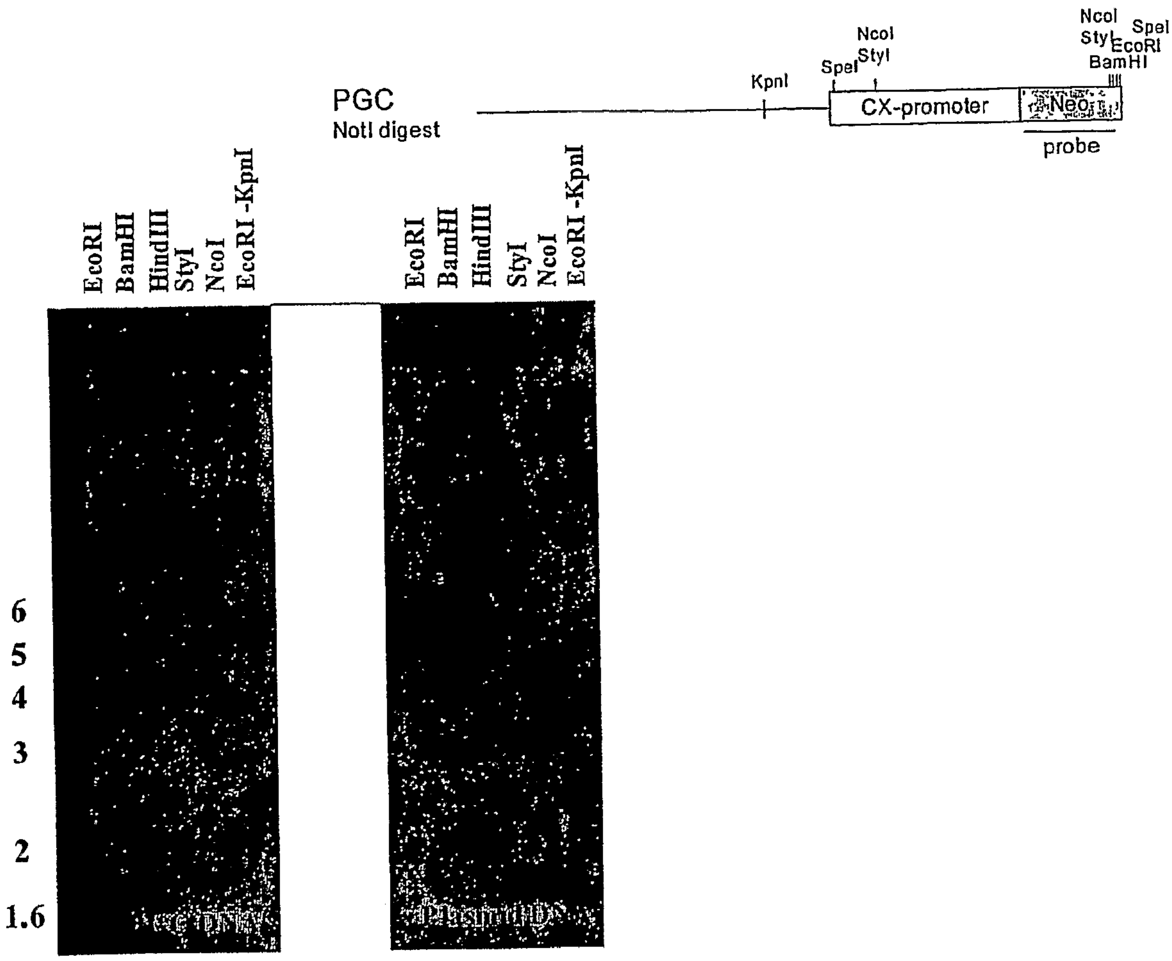
PGC
NotI digest
KpnI
SpeI
NcoI
StyI
CX-promoter
Neo
NcoI
StyI
SpeI
EcoRI
BamHI
probe
EcoRI
BamHI
HindIII
StyI
NcoI
EcoRI -KpnI
EcoRI
BamHI
HindIII
StyI
NcoI
EcoRI -KpnI
6
5
4
3
2
1.6

FIGURE 8

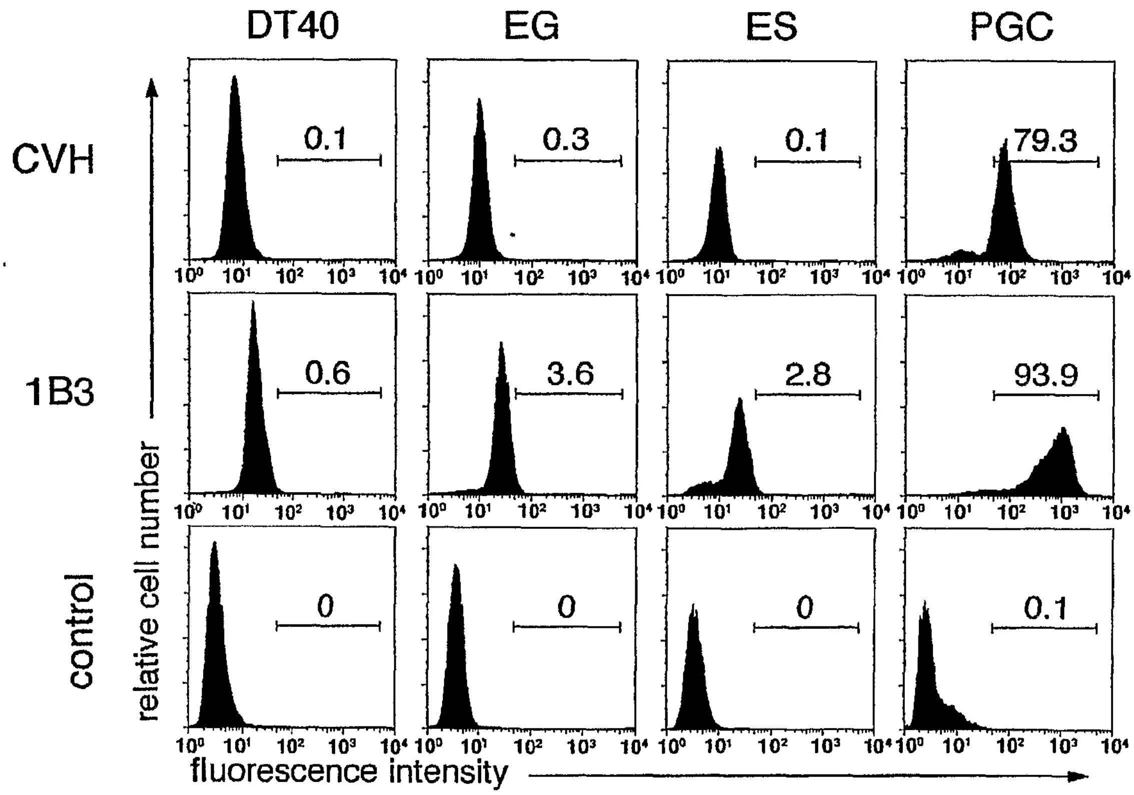
DT40
EG
ES
PGC
CVH
1B3
control
relative cell number
fluorescence intensity
0.1
0.3
0.1
79.3
0.6
3.6
2.8
93.9
0
0
0
0.1

FIGURE 9

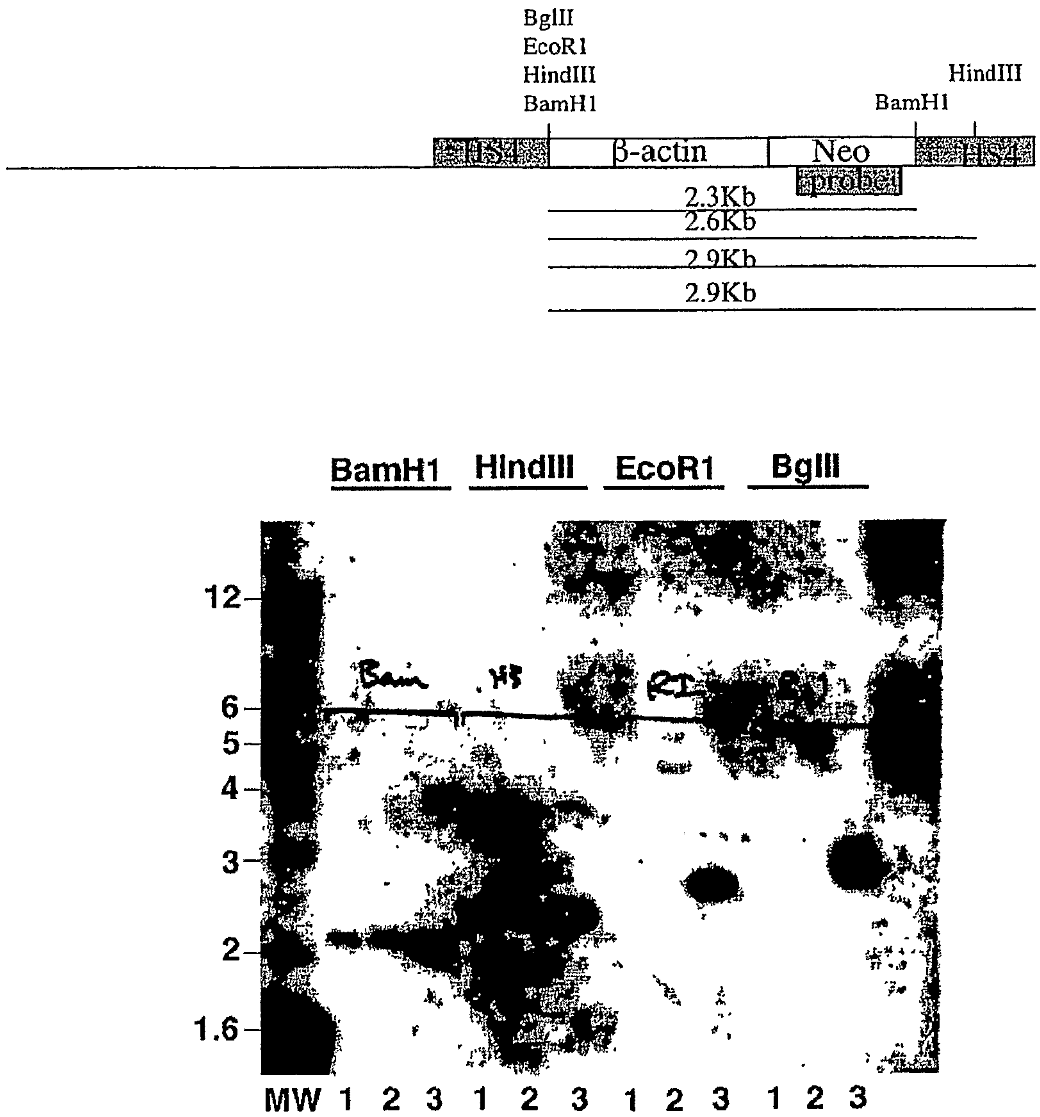
BglII
EcoR1
HindIII
BamH1
HindIII
BamH1
HS4
β-actin
Neo
HS4
probe
2.3Kb
2.6Kb
2.9Kb
2.9Kb
BamH1
HindIII
EcoR1
BglII
12
6
5
4
3
2
1.6
MW 1 2 3 1 2 3 1 2 3 1 2 3

FIGURE 10

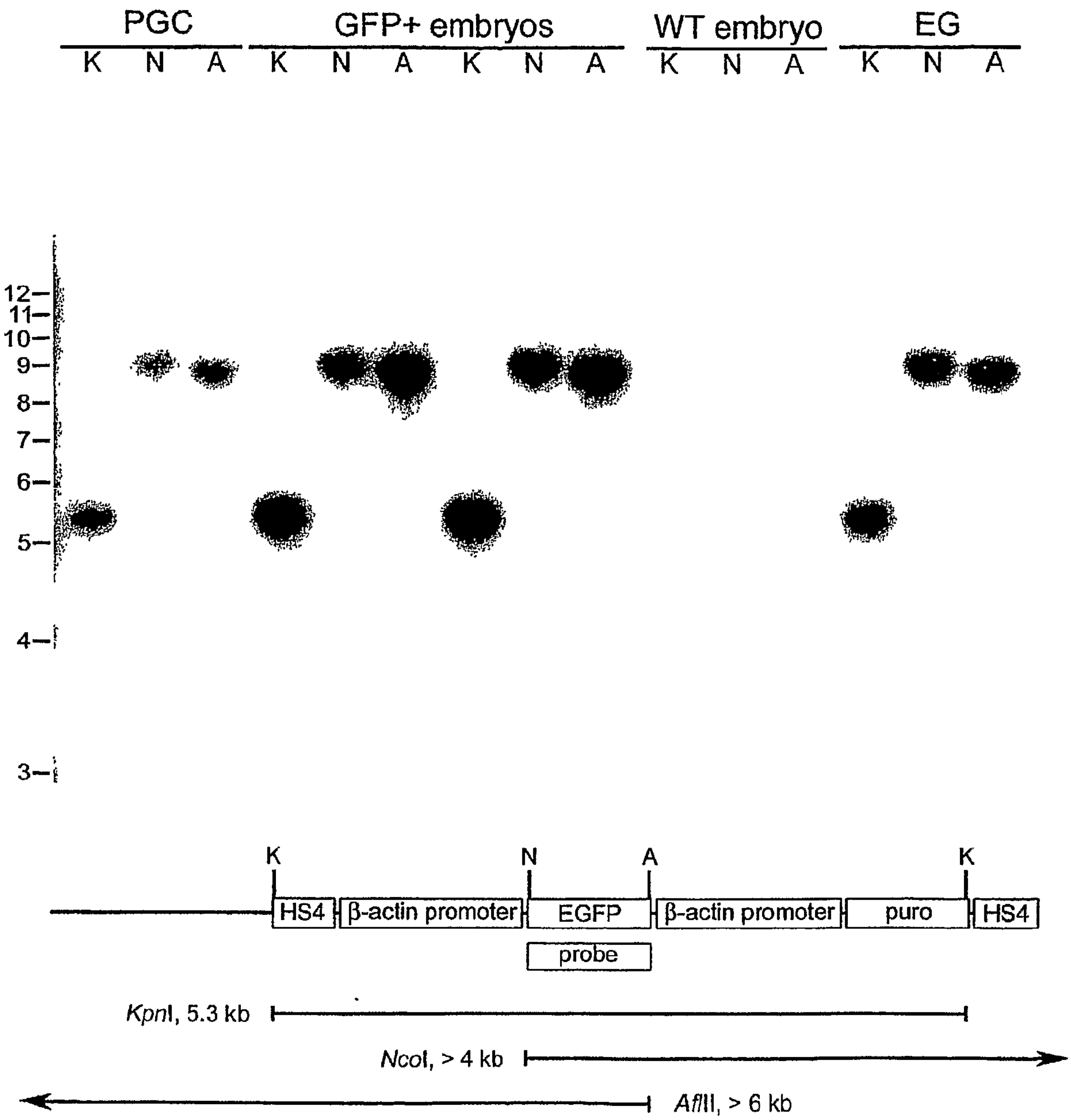

PGC
GFP+ embryos
WT embryo
EG
K N A K N A K N A K N A K N A
12
11
10
9
8
7
6
5
4
3
K
N
A
K
HS4
β-actin promoter
EGFP
β-actin promoter
puro
HS4
probe
KpnI, 5.3 kb
NcoI, > 4 kb
AflII, > 6 kb


FIGURE 11

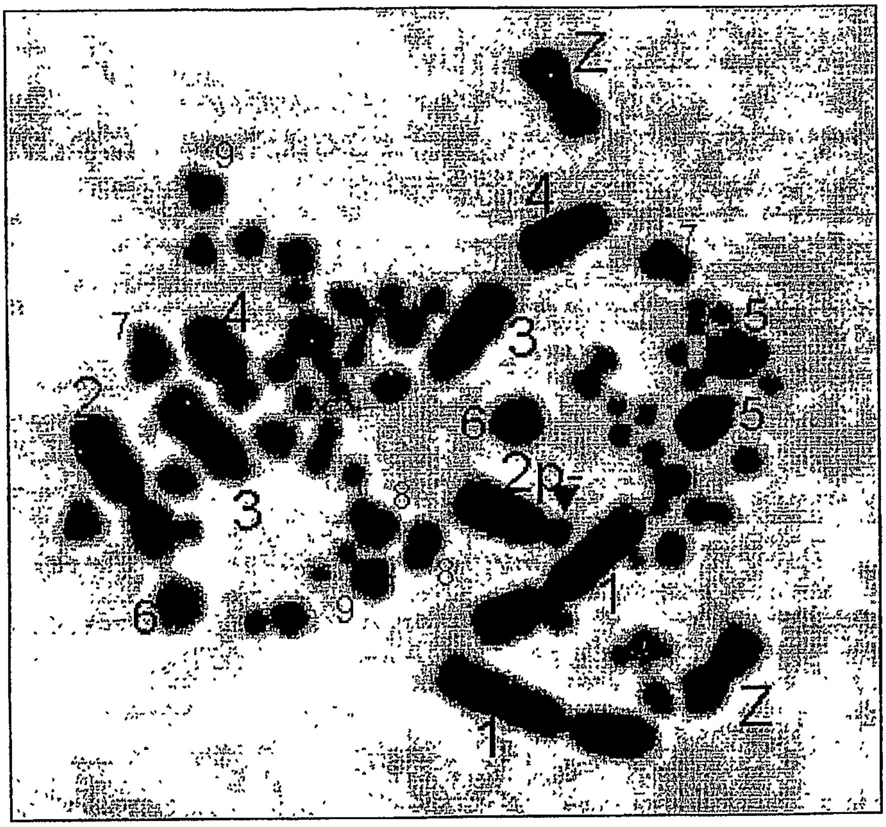


FIGURE 12

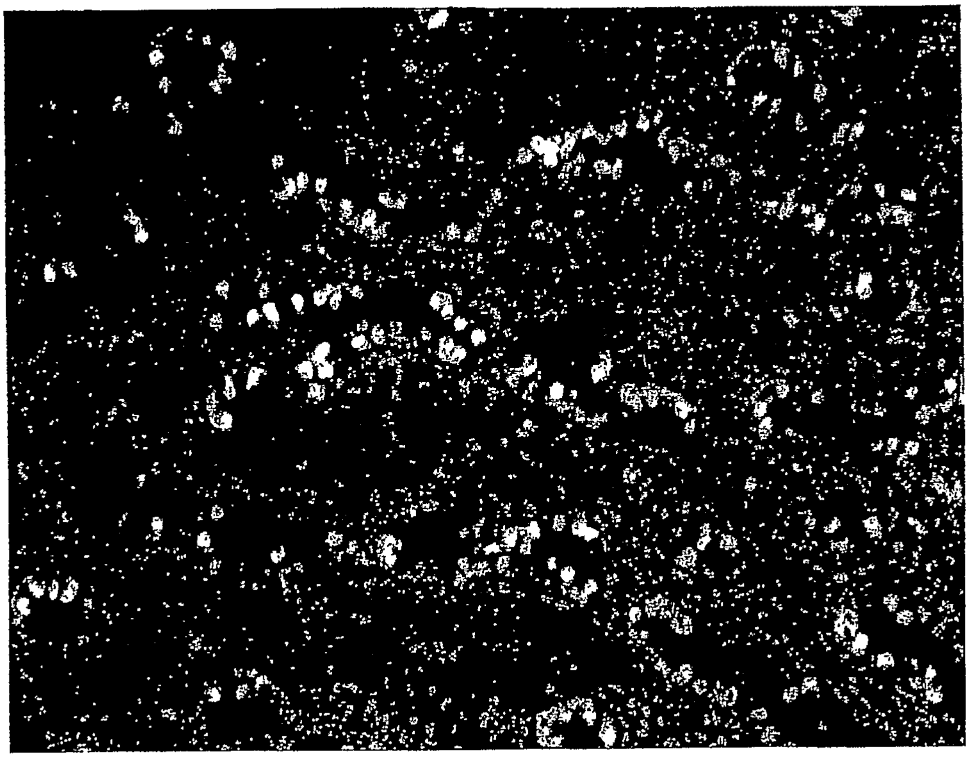

FIGURE 13

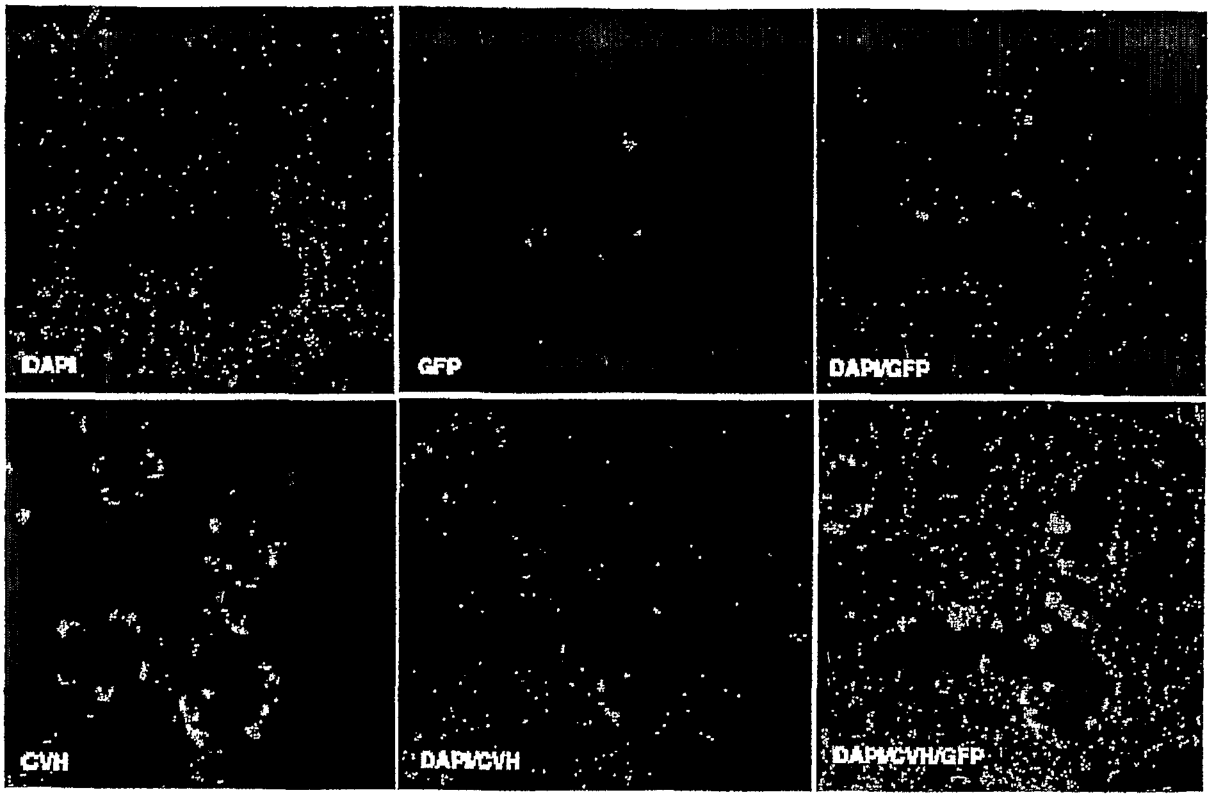


FIGURE 14

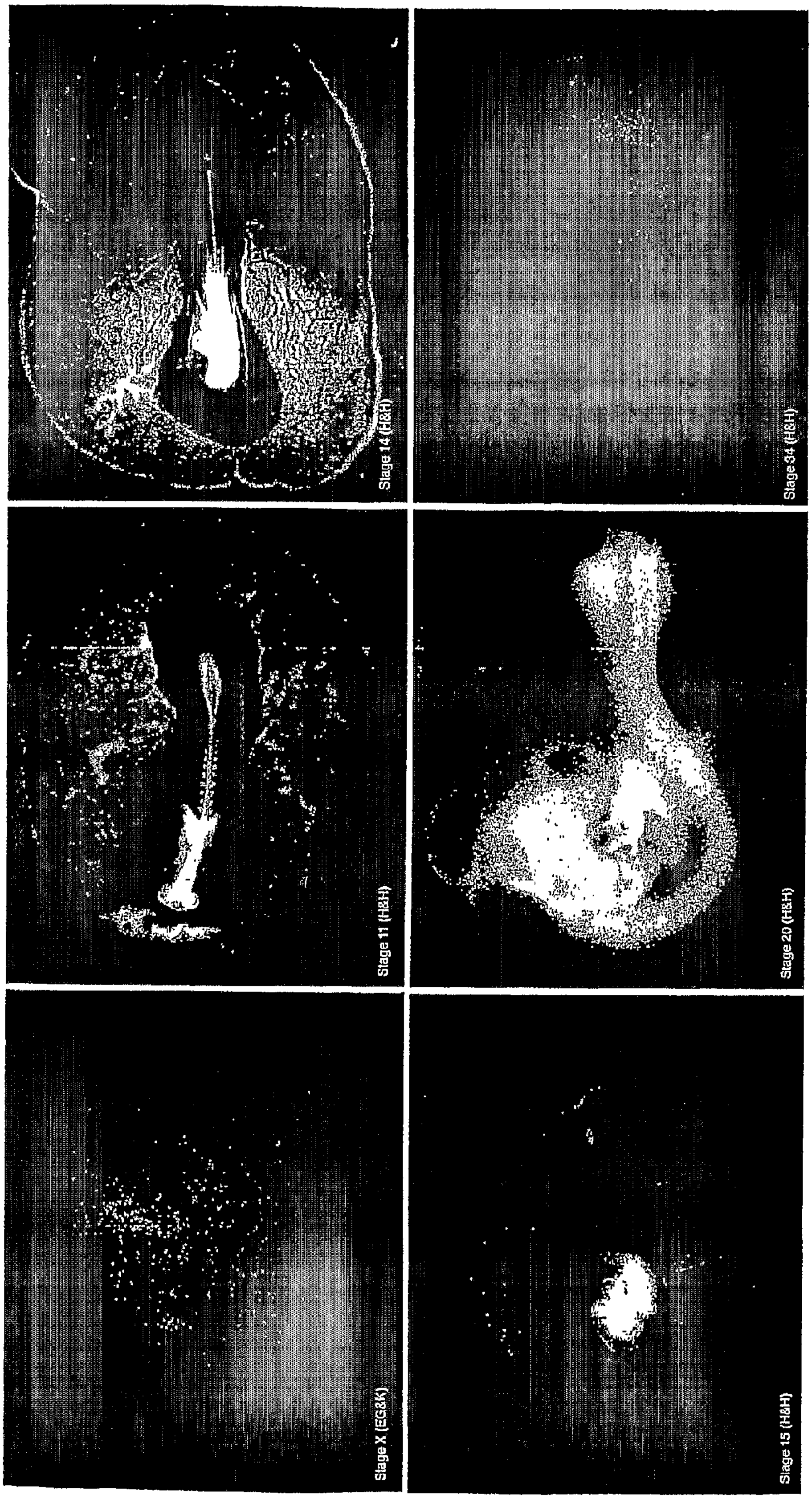


FIGURE 15

SUBSTITUTE SHEET (RULE 26)

FIGURE 16

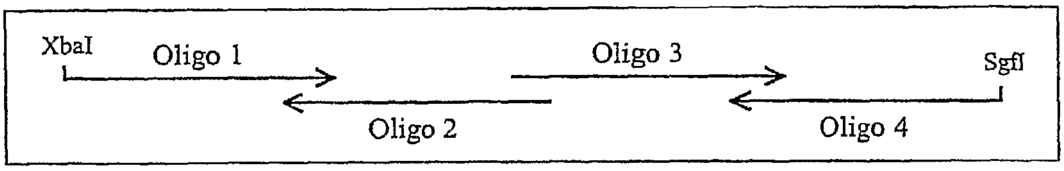

FIGURE 17

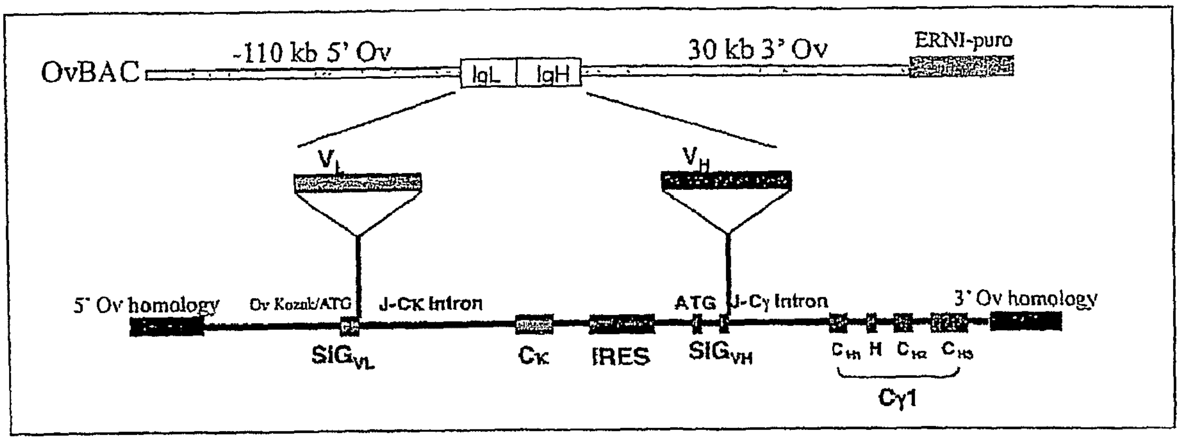
OvBAC
~110 kb 5' Ov
IgL
IgH
30 kb 3' Ov
ERNI-puro
$V_L$
$V_H$
5' Ov homology
Ov Kozak/ATG
J-Cκ Intron
ATG
J-Cγ Intron
3' Ov homology
$SIG_{VL}$
Cκ
IRES
$SIG_{VH}$
$C_{H1}$ H $C_{H2}$ $C_{H3}$
Cγ1

FIGURE 18

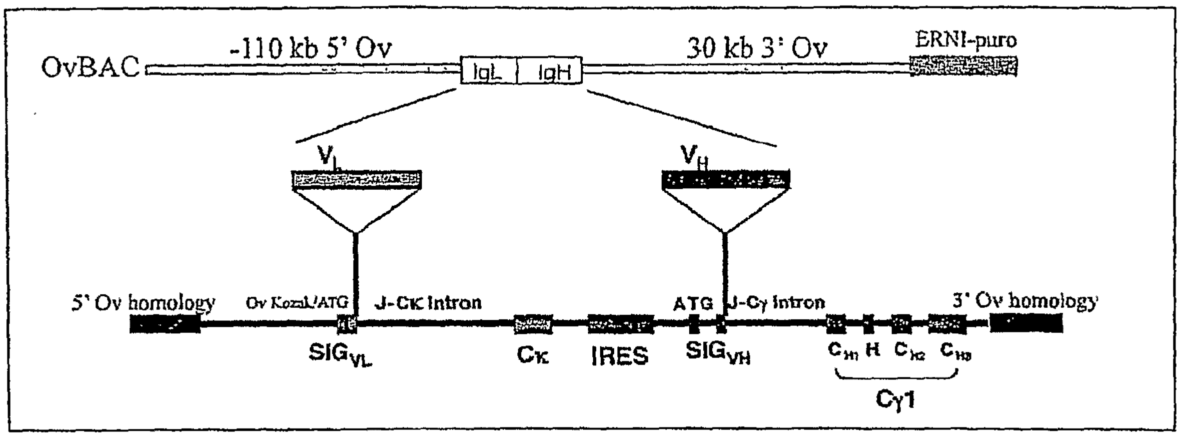
OvBAC
-110 kb 5' Ov
IgL
IgH
30 kb 3' Ov
ERNI-puro
$V_L$
$V_H$
5' Ov homology
Ov Kozak/ATG
J-Cκ Intron
ATG
J-Cγ Intron
3' Ov homology
$SIG_{VL}$
Cκ
IRES
$SIG_{VH}$
$C_{H1}$ H $C_{H2}$ $C_{H3}$
Cγ1

Europäisches Patentamt
European Patent Office
Office européen des brevets

# EUROPEAN SEARCH REPORT

Application Number
EP 16 17 4967

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 02/067669 A2 (TRANXENOGEN INC [US]; BAGUISI ALEXANDER [US]; EBERT KARL M [US]) 6 September 2002 (2002-09-06)<br>* examples 3, 4 *<br>----- | 1-8 | INV.<br>A01K67/027<br>C12N15/00<br>C12N5/0735<br>C12N15/85 |
| A | YEONG HO HONG ET AL: "Improved transfection efficiency of chicken gonadal primordial germ cells for the production of transgenic poultry",<br>TRANSGENIC RESEARCH, LONDON, GB LNKD-DOI:10.1023/A:1008861826681,<br>vol. 7, no. 4, 1 July 1998 (1998-07-01), pages 247-252, XP001058413,<br>ISSN: 0962-8819<br>* page 248, left-hand column, last paragraph - page 249, paragraph 1 *<br>* page 250, paragraph 1 *<br>* figure 4 *<br>----- | 1-8 | |
| A | WO 2004/067713 A2 (AVIGENICS INC [US]; UNIV GEORGIA [US]; IVARIE ROBERT D [US]; HARVEY AL) 12 August 2004 (2004-08-12)<br>* paragraph [0085] *<br>----- | 1-8 | TECHNICAL FIELDS SEARCHED (IPC)<br>A01K<br>C12N |
| A | PETITTE J N ET AL: "Avian pluripotent stem cells",<br>MECHANISMS OF DEVELOPMENT, ELSEVIER SCIENCE IRELAND LTD, IE LNKD-DOI:10.1016/J.MOD.2004.05.003,<br>vol. 121, no. 9,<br>1 September 2004 (2004-09-01), pages 1159-1168, XP009077049,<br>ISSN: 0925-4773<br>* page 1162, right-hand column, paragraph 1 *<br>-----<br>-/-- | 1-8 | |

The present search report has been drawn up for all claims

1

| Place of search | Date of completion of the search | Examiner |
|---|---|---|
| The Hague | 27 September 2016 | Brouns, Gaby |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 17 4967

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SANG HELEN ED - WITTBRODT JOACHIM ET AL: "Prospects for transgenesis in the chick", MECHANISMS OF DEVELOPMENT, ELSEVIER SCIENCE IRELAND LTD, IE, vol. 121, no. 9, 1 September 2004 (2004-09-01), pages 1179-1186, XP002341506, ISSN: 0925-4773, DOI: 10.1016/J.MOD.2004.05.012 ----- | 1-8 | |
| A,P | GASZNER MIKLOS ET AL: "Insulators: exploiting transcriptional and epigenetic mechanisms", NATURE REVIEWS GENETICS, MACMILLAN MAGAZINES, GB LNKD- DOI:10.1038/NRG1925, vol. 7, no. 9, 1 September 2006 (2006-09-01), pages 703-710, XP009105578, * abstract * ----- | 1-8 | |
| T | LEIGHTON PHILIP A ET AL: "Genetic modification of primordial germ cells by gene trapping, gene targeting, and phi C31 integrase", MOLECULAR REPRODUCTION AND DEVELOPMENT, vol. 75, no. 7, July 2008 (2008-07), pages 1163-1175, XP002604337, ISSN: 1040-452X ----- | | TECHNICAL FIELDS SEARCHED (IPC) |
| T | VAN DE LAVOIR MARIE-CECILE ET AL: "Germline transmission of genetically modified primordial germ cells", NATURE, NATURE PUBLISHING GROUP, UNITED KINGDOM, vol. 441, no. 7094, 1 June 2006 (2006-06-01), pages 766-769, XP009093457, ISSN: 0028-0836, DOI: 10.1038/NATURE04831 ----- -/-- | | |

The present search report has been drawn up for all claims

1

| Place of search | Date of completion of the search | Examiner |
|---|---|---|
| The Hague | 27 September 2016 | Brouns, Gaby |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 17 4967

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | SHIUE Y-L ET AL: "Establishment of the Long-Term In Vitro Culture System for Chicken Primordial Germ Cells", REPRODUCTION IN DOMESTIC ANIMALS, vol. 44, no. 1, February 2009 (2009-02), pages 55-61, XP002604409, ISSN: 0936-6768 * page 59, right-hand column, paragraph 3 - page 60, left-hand column * ----- | | |
| T | J. Kureczka: "Origen publishes in Nature a robust and versatile method for creating trangenic chickens", , 7 June 2006 (2006-06-07), XP002604336, Retrieved from the Internet: URL:http://www.eurekalert.org/pub_releases/2006-06/ka-opi060506.php [retrieved on 2010-10-04] * paragraph [0002] * ----- | | |

TECHNICAL FIELDS SEARCHED (IPC)

The present search report has been drawn up for all claims

1

| Place of search | Date of completion of the search | Examiner |
|---|---|---|
| The Hague | 27 September 2016 | Brouns, Gaby |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 17 4967

This annex lists the patent family members relating to the patent documents cited in the above-mentioned European search report. The members are as contained in the European Patent Office EDP file on
The European Patent Office is in no way liable for these particulars which are merely given for the purpose of information.

27-09-2016

| Patent document cited in search report | Publication date | Patent family member(s) | Publication date |
|---|---|---|---|
| WO 02067669 A2 | 06-09-2002 | CA 2438612 A1 | 06-09-2002 |
| | | EP 1361788 A2 | 19-11-2003 |
| | | JP 2005507234 A | 17-03-2005 |
| | | WO 02067669 A2 | 06-09-2002 |
| WO 2004067713 A2 | 12-08-2004 | CN 102660581 A | 12-09-2012 |
| | | EP 1592773 A2 | 09-11-2005 |
| | | US 2004019923 A1 | 29-01-2004 |
| | | US 2005188430 A1 | 25-08-2005 |
| | | US 2005188431 A1 | 25-08-2005 |
| | | US 2012266265 A1 | 18-10-2012 |
| | | WO 2004067713 A2 | 12-08-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 0325270 W
- WO 04015123 A, Zhu
- WO 0047717 A
- WO 9906533 A
- WO 9906534 A
- WO 0111019 A
- US 4959317 A
- US 6130364 A
- US 6091001 A
- US 5985614 A
- WO 5585089 A
- US 049229 A


### Non-patent literature cited in the description


- **KLOBUTCHER, L.A. ; F.H. RUDDLE.** *Annu. Rev. Biochem.,* 1981, vol. 50, 533-554
- **FOUMIER, R.E. ; F.H. RUDDLE.** *Proc. Natl. Acad. Sci. U.S.A.,* 1977, vol. 74, 319-323
- **TAJIMA et al.** *Theriogenology,* 1993, vol. 40, 509-519
- **NAITO et al.** *Mol. Reprod. Dev.,* 1994, vol. 39, 153-161
- **NAITO et al.** *J Reprod. Fert.,* 1999, vol. 117, 291-298
- **KAGAMI et al.** *Mol Reprod Dev,* 1997, vol. 48, 501-510
- **PETITTE.** *J Poultry Sci,* 2002, vol. 39, 205-228
- **HAMBURGER ; HAMILTON.** *J Morph,* 1951, vol. 88, 49-92
- **SWIFT.** *Am. J. Anat.,* 1914, vol. 15, 483-516
- **MEYER.** *Dev Biol.,* 1964, vol. 10, 154-190
- **FUJIMOTO et al.** *Anat. Rec.,* 1976, vol. 185, 139-154
- **PARK et al.** *Mol. Reprod. Dev.,* 2003, vol. 65, 389-395
- **PARK ; HAN.** *Mol. Reprod. Dev.,* 2000, vol. 56, 475-482
- **CHANG et al.** *Cell Biology International,* 1997, vol. 21, 495-499
- **CHANG et al.** *Cell Biology International,* 1995, vol. 19, 569-576
- **PARK et al.** *Biol. Reprod.,* 2003, vol. 68, 1657-1662
- **HAN et al.** *Theriogenology,* 2002, vol. 58, 1531-1539
- **VICK et al.** *Proc. R. Soc. Lond. B,* 1993, vol. 251, 179-182
- **SANG et al.** It is unlikely that PGCs can be maintained in culture and proliferate for the extended period necessary to identify gene targeting events without losing their ability to migrate to the developing gonad after transfer. *Prospects for Transgenesis in the Chick, Mechanisms of Development,* 2004, vol. 121, 1179-1186
- **KLOBUTCHER, L.A. ; F.H. RUDDLE.** *Ann. Rev. Biochem.,* 1981, vol. 50, 533-554
- **FOUMIER, R.E. ; RUDDLE, F.H.** *Proc. Natl. Acad. Sci.,* 1977, vol. 74, 319-323
- **REYNAUD.** *Bull Soc. Zool. Francaise,* 1977, vol. 102, 417-429
- **REYNAUD.** *Arch Anat. Micro. Morph. Exp.,* 1981, vol. 70, 251-258
- **AIGE-GIL ; SIMKISS.** *Res. Vet. Sci.,* 1991, vol. 50, 139-144
- **VICK et al.** *J. Reprod. Fert.,* 1993, vol. 98, 637-641
- **BRESLER et al.** *Brit. Poultry Sci.,* 1994, vol. 35, 241-247
- **VAN DE LAVOIR ; 2006 et al.** High Grade Somatic Chimeras from Chicken Embryonic Stem Cells. *Mechanisms of Development,* vol. 12, 31-41
- **VAN DE LAVOIR ; MATHER-LOVE.** Chicken Embryonic Stem Cells; Culture and Chimera Production. *Methods in Enzymology,* 2006
- **THOMAS, K.R. ; M.R. CAPECCHI.** *Cell,* 1987, vol. 51, 503-512
- **WALDMAN, A.S.** *Crit. Rev. Oncol. Hematol.,* 1992, vol. 12, 49-64
- **JAKOBOVITS, A.** *Curr. Biol.,* 1994, vol. 4, 761-763
- **SMITH, A.J. et al.** *Nat. Genet.,* 1995, vol. 9, 376-385
- **RAMIREZ-SOLIS, R. et al.** *Nature,* 1995, vol. 378, 720-724

- **HALFTER, W. ; SCHURER, B. ; HASSELHOM, H. M. ; CHRIST, B. ; GIMPEL, E. ; EPPERLEIN, H. H.** An ovomucin-like protein on the surface of migrating primordial germ cells of the chick and rat. *Development,* 1996, vol. 122, 915-23
- **NIWA, H. ; YAMAMURA, K. ; MIYAZAKI, J.** Efficient selection for high-expression transfectants with a novel eukaryotic vector. *Gene,* 1991, vol. 108, 193-9
- **BURGESS-BEUSSE, B. ; FARRELL, C. ; GASZNER, M. ; LITT, M. ; MUTSKOV, V. ; RECILLAS-TARGA, F. ; SIMPSON, M. ; WEST, A. ; FELSENFELD, G.** The insulation of genes from external enhancers and silencing chromatin. *Proc.. Natl. Acad. Sci. USA,* 2002, vol. 99 (4), 16433-7
- **STREIT, A. ; BERLINER, A. J. ; PAPANAYOTOU, C. ; SIRULNIK, A. ; STEM, C. D.** Initiation of neural induction by FGF signalling before gastrulation. *Nature,* 2000, vol. 406, 74-8
- **ACLOQUE, H. ; RISSON, V. ; BIROT, A. ; KUNITA, R. ; PAIN, B. ; SAMARUT, J.** Identification of a new gene family specifically expressed in chicken embryonic stem cells and early embryo. *Mech Dev,* 2001, vol. 103, 79-91
- **COPELAND, N. G. ; JENKINS, N. A. ; COURT, D. L.** Recombineering: a powerful new tool for mouse functional genomics. *Nat Rev Genet,* 2001, vol. 2, 769-79
- **WANG, Z. ; ENGLER, P. ; LONGACRE, A. ; STORB, U.** An efficient method for high-fidelity BAC/PAC retrofitting with a selectable marker for mammalian cell transfection. *Genome Res.,* 2001, vol. 11, 137-42
- **ZHU, L. et al.** *Nat. Biotech.,* 2005, vol. 23, 1159-1169
- **DILLMAN.** *Cancer Biother. & Radiopharm.,* 1997, vol. 12, 223-225
- **DEO et al.** *Immunology Today,* 1997, vol. 18, 127